# EUROPEAN PATENT SPECIFICATION

(11) **EP 4 221 568 B1**
(45) Date of publication and mention of the grant of the patent: **09.09.2026**
(21) Application number: 21786589.8
(22) Date of filing: 28.09.2021
(51) Int. Cl.: A61B 5/00, A61B 5/0205, A61M 16/00

(54) **SYSTEM AND METHOD FOR DETERMINING USAGE OF A RESPIRATORY THERAPY SYSTEM**
SYSTEM UND VERFAHREN ZUR BESTIMMUNG DER VERWENDUNG EINES ATEMTHERAPIESYSTEMS
SYSTÈME ET PROCÉDÉ POUR DÉTERMINER L'USAGE D'UN SYSTÈME DE THÉRAPIE RESPIRATOIRE

(30) Priority: 29.09.2020 US 202063084978 P
(43) Date of publication of application: 09.08.2023
(73) Proprietor: ResMed Sensor Technologies Limited, Sandyford D18 T6T7 Dublin (IE)
(72) Inventor: DODD, Stephen, Dublin, D18 T6T7 (IE); SHOULDICE, Redmond, Dublin, D18 T6T7 (IE); COSTELLO, Michael John, Dublin, D18 T6T7 (IE); LYON, Graeme Alexander, Dublin, D18 T6T7 (IE)
(74) Representative: Mathys & Squire
(86) International application number: PCT/IB2021/058813
(87) International publication number: WO 2022/070022

(56) References cited:
- WO-A1-2019/122412
- WO-A1-2019/152699
- US-A1- 2005 061 319
- US-A1- 2014 005 502
- C.W. CHAN, C.S. CHAN, K. SACHINDHELM, S. FARRUGIA: "Central Sleep Apnea detection: an algorithm based on detection of cardiogenic oscillations on an auirflow signal", AUSTRALASIAN PHYSICAL AND ENGINEERING SCIENCES IN MEDICINE, MEDICINE, MELBOURNE, AU; ABSTRACTS, vol. 27, no. 4, 1 December 2004 (2004-12-01), pages 237 - 342, XP037138593, ISSN: 0158-9938, DOI: 10.1007/BF03178657

## Description

### CROSS-REFERENCE TO RELATED APPLICATIONS

This application claims the benefit of and priority to U.S. Provisional Patent Application No. 63/084,978, filed September 29, 2020.

### TECHNICAL FIELD

The present disclosure relates generally to systems and methods for determining whether a user is using a respiratory therapy system, and more particularly, to systems and methods for distinguishing between the user using the respiratory therapy system and not using the respiratory therapy system and quantifying physiological effects of using and not using the respiratory therapy system.

### BACKGROUND

Many individuals suffer from sleep-related and/or respiratory disorders such as, for example, Periodic Limb Movement Disorder (PLMD), Restless Leg Syndrome (RLS), Sleep-Disordered Breathing (SDB) such as Obstructive Sleep Apnea (OSA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), chest wall disorders, and insomnia. Many of these disorders can be treated using a respiratory therapy system, while others may be treated using a different technique. However, some users find such systems to be uncomfortable, difficult to use, expensive, aesthetically unappealing and/or fail to perceive the benefits associated with using the system. As a result, some users may elect not to use the respiratory therapy system diligently, absent a demonstration of the severity of their symptoms when respiratory therapy treatment is not used and/or benefits to their symptoms when respiratory therapy treatment is used. Quantifying some benefits of the respiratory therapy system can help improve diligence. The present disclosure is directed to solving these and other problems.

### SUMMARY

According to some implementations of the present disclosure, a system includes a respiratory therapy system, a sensor configured to generate physiological data associated with a user of the respiratory therapy system during a sleep session, a memory storing machine-readable instructions, and an electronic device. The electronic device includes one or more processors configured to execute the machine-readable instructions to process the generated physiological data to distinguish between on-therapy data and off-therapy data. The on-therapy data is the generated physiological data while the respiratory therapy system is coupled to the user and supplies pressurized air to an airway of the user. The off-therapy data is the generated physiological data while the respiratory therapy system is not supplying pressurized air to the airway of the user. The electronic device is further configured to execute the machine-readable instruction to determine a sleep measure based at least in part on the off-therapy data.

According to some implementations of the present disclosure, a method includes generating, by a sensor, physiological data associated with a user during a sleep session. The method also includes processing, by an electronic device including one or more processors, the generated physiological data to distinguish between on-therapy data and off-therapy data. The on-therapy data is the generated physiological data while a respiratory therapy system is coupled to the user and supplies pressurized air to an airway of the user. The off-therapy data is the generated physiological data while the respiratory therapy system is not supplying pressurized air to the airway of the user. The method also includes determining, by the electronic device, a sleep measure based at least in part on the off-therapy data.

According to some implementations of the present disclosure, an electronic device includes a memory storing machine-readable instructions and a control system including one or more processors configured to execute the machine-readable instructions to: cause a sensor to generate physiological data associated with a user during a sleep session; process the generated physiological data to determine on-therapy data and off-therapy data, wherein (i) the on-therapy data is the generated physiological data while a respiratory therapy system, coupled to the user, supplies pressurized air to an airway of the user, and (ii) the off-therapy data is the generated physiological data while the respiratory therapy system is not providing pressurized air to the airway of the user; and determine a sleep measure based at least in part on the off-therapy data.

A method and a system according to the invention are defined in the independent claims.

The above summary is not intended to represent each implementation or every aspect of the present disclosure. Additional features and benefits of the present disclosure are apparent from the detailed description and figures set forth below.

### BRIEF DESCRIPTION OF THE DRAWINGS

FIG. 1 is a functional block diagram of a system, according to some implementations of the present disclosure;
FIG. 2 is a perspective view of at least a portion of the system of FIG. 1, a user, and a bed partner, according to some implementations of the present disclosure;
FIG. 3 illustrates an exemplary timeline for a sleep session, according to some implementations of the present disclosure;
FIG. 4 illustrates an exemplary hypnogram associated with the sleep session of FIG. 3, according to some implementations of the present disclosure;
FIG. 5 is a process flow diagram for a method for determining a sleep measure, according to some implementations of the present disclosure;
FIG. 6A illustrates audio artifacts for distinguishing between on and off therapy, according to some implementations of the present disclosure;
FIG. 6B illustrates another example of audio artifacts for distinguishing between on and off therapy, according to some implementations of the present disclosure;
FIG. 7 illustrates a mapping of events, according to some implementations of the present disclosure; and
FIG. 8 illustrates a histogram and events graph, according to some implementations of the present disclosure.

While the present disclosure is susceptible to various modifications and alternative forms, specific implementations and embodiments thereof have been shown by way of example in the drawings and will herein be described in detail. It should be understood, however, that it is not intended to limit the present disclosure to the particular forms disclosed, but on the contrary, the present disclosure is to cover all modifications, equivalents, and alternatives falling within the scope of the present disclosure as defined by the appended claims.

### DETAILED DESCRIPTION

Many individuals suffer from sleep-related and/or respiratory disorders. Examples of sleep-related and/or respiratory disorders include Periodic Limb Movement Disorder (PLMD), Restless Leg Syndrome (RLS), Sleep-Disordered Breathing (SDB) such as Obstructive Sleep Apnea (OSA), Central Sleep Apnea (CSA), and other types of apneas such as mixed apneas and hypopneas, Respiratory Effort Related Arousal (RERA), Cheyne-Stokes Respiration (CSR), respiratory insufficiency, Obesity Hyperventilation Syndrome (OHS), Chronic Obstructive Pulmonary Disease (COPD), Neuromuscular Disease (NMD), rapid eye movement (REM) behavior disorder (also referred to as RBD), dream enactment behavior (DEB), hypertension, diabetes, stroke, insomnia, and chest wall disorders.

Obstructive Sleep Apnea (OSA) is a form of Sleep Disordered Breathing (SDB), and is characterized by events including occlusion or obstruction of the upper air passage during sleep resulting from a combination of an abnormally small upper airway and the normal loss of muscle tone in the region of the tongue, soft palate and posterior oropharyngeal wall. More generally, an apnea generally refers to the cessation of breathing caused by blockage of the air (Obstructive Sleep Apnea) or the stopping of the breathing function (often referred to as Central Sleep Apnea). Typically, the individual will stop breathing for between about 15 seconds and about 30 seconds during an obstructive sleep apnea event.

Other types of apneas include hypopnea, hyperpnea, and hypercapnia. Hypopnea is generally characterized by slow or shallow breathing caused by a narrowed airway, as opposed to a blocked airway. Hyperpnea is generally characterized by an increase depth and/or rate of breathing. Hypercapnia is generally characterized by elevated or excessive carbon dioxide in the bloodstream, typically caused by inadequate respiration.

Cheyne-Stokes Respiration (CSR) is another form of sleep disordered breathing. CSR is a disorder of a patient's respiratory controller in which there are rhythmic alternating periods of waxing and waning ventilation known as CSR cycles. CSR is characterized by repetitive deoxygenation and re-oxygenation of the arterial blood.

Obesity Hyperventilation Syndrome (OHS) is defined as the combination of severe obesity and awake chronic hypercapnia, in the absence of other known causes for hypoventilation. Symptoms include dyspnea, morning headache and excessive daytime sleepiness.

Chronic Obstructive Pulmonary Disease (COPD) encompasses any of a group of lower airway diseases that have certain characteristics in common, such as increased resistance to air movement, extended expiratory phase of respiration, and loss of the normal elasticity of the lung.

Neuromuscular Disease (NMD) encompasses many diseases and ailments that impair the functioning of the muscles either directly via intrinsic muscle pathology, or indirectly via nerve pathology. Chest wall disorders are a group of thoracic deformities that result in inefficient coupling between the respiratory muscles and the thoracic cage.

A Respiratory Effort Related Arousal (RERA) event is typically characterized by an increased respiratory effort for ten seconds or longer leading to arousal from sleep and which does not fulfill the criteria for an apnea or hypopnea event. RERAs are defined as a sequence of breaths characterized by increasing respiratory effort leading to an arousal from sleep, but which does not meet criteria for an apnea or hypopnea. These events must fulfil both of the following criteria: (1) a pattern of progressively more negative esophageal pressure, terminated by a sudden change in pressure to a less negative level and an arousal, and (2) the event lasts ten seconds or longer. In some implementations, a Nasal Cannula/Pressure Transducer System is adequate and reliable in the detection of RERAs. A RERA detector may be based on a real flow signal derived from a respiratory therapy device. For example, a flow limitation measure may be determined based on a flow signal. A measure of arousal may then be derived as a function of the flow limitation measure and a measure of sudden increase in ventilation. One such method is described in WO 2008/138040 and U.S. Patent No. 9,358,353, assigned to ResMed Ltd.,

These and other disorders are characterized by particular events (e.g., snoring, an apnea, a hypopnea, a restless leg, a sleeping disorder, choking, an increased heart rate, labored breathing, an asthma attack, an epileptic episode, a seizure, or any combination thereof) that can occur when the individual is sleeping.

The Apnea-Hypopnea Index (AHI) is an index used to indicate the severity of sleep apnea during a sleep session. The AHI is calculated by dividing the number of apnea and/or hypopnea events experienced by the user during the sleep session by the total number of hours of sleep in the sleep session. The event can be, for example, a pause in breathing that lasts for at least 10 seconds. An AHI that is less than 5 is considered normal. An AHI that is greater than or equal to 5, but less than 15 is considered indicative of mild sleep apnea. An AHI that is greater than or equal to 15, but less than 30 is considered indicative of moderate sleep apnea. An AHI that is greater than or equal to 30 is considered indicative of severe sleep apnea. In children, an AHI that is greater than 1 is considered abnormal. Sleep apnea can be considered "controlled" when the AHI is normal, or when the AHI is normal or mild. The AHI can also be used in combination with oxygen desaturation levels to indicate the severity of Obstructive Sleep Apnea.

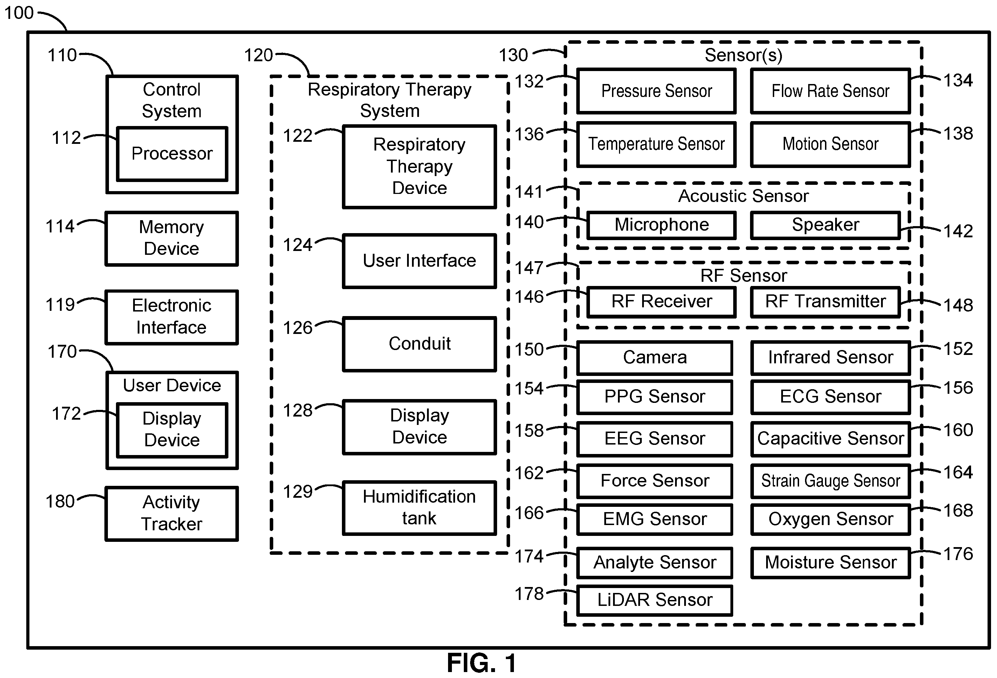

Referring to FIG. 1, a system 100, according to some implementations of the present disclosure, is illustrated. The system 100 includes a control system 110, a memory device 114, an electronic interface 119, one or more sensors 130, and one or more user devices 170. In some implementations, the system 100 further optionally includes a respiratory therapy system 120, an activity tracker 180, or any combination thereof.

The control system 110 includes one or more processors 112 (hereinafter, processor 112). The control system 110 is generally used to control (e.g., actuate) the various components of the system 100 and/or analyze data obtained and/or generated by the components of the system 100. The processor 112 can be a general or special purpose processor or microprocessor. While one processor 112 is shown in FIG. 1, the control system 110 can include any suitable number of processors (e.g., one processor, two processors, five processors, ten processors, etc.) that can be in a single housing, or located remotely from each other. The control system 110 can be coupled to and/or positioned within, for example, a housing of the user device 170, a portion (e.g., a housing) of the respiratory therapy system 120, and/or within a housing of one or more of the sensors 130. The control system 110 can be centralized (within one such housing) or decentralized (within two or more of such housings, which are physically distinct). In such implementations including two or more housings containing the control system 110, such housings can be located proximately and/or remotely from each other.

The memory device 114 stores machine-readable instructions that are executable by the processor 112 of the control system 110. The memory device 114 can be any suitable computer readable storage device or media, such as, for example, a random or serial access memory device, a hard drive, a solid state drive, a flash memory device, etc. While one memory device 114 is shown in FIG. 1, the system 100 can include any suitable number of memory devices 114 (e.g., one memory device, two memory devices, five memory devices, ten memory devices, etc.). The memory device 114 can be coupled to and/or positioned within a housing of the respiratory therapy device 122, within a housing of the user device 170, within a housing of one or more of the sensors 130, or any combination thereof. Like the control system 110, the memory device 114 can be centralized (within one such housing) or decentralized (within two or more of such housings, which are physically distinct).

In some implementations, the memory device 114 (FIG. 1) stores a user profile associated with the user. The user profile can include, for example, demographic information associated with the user, biometric information associated with the user, medical information associated with the user, self-reported user feedback, sleep parameters associated with the user (e.g., sleep-related parameters recorded from one or more earlier sleep sessions), or any combination thereof. The demographic information can include, for example, information indicative of an age of the user, a gender of the user, a race of the user, a geographic location of the user, a relationship status, a family history of insomnia, an employment status of the user, an educational status of the user, a socioeconomic status of the user, or any combination thereof. The medical information can include, for example, including indicative of one or more medical conditions associated with the user, medication usage by the user, or both. The medical information data can further include a multiple sleep latency test (MSLT) test result or score and/or a Pittsburgh Sleep Quality Index (PSQI) score or value. The self-reported user feedback can include information indicative of a self-reported subjective sleep score (e.g., poor, average, excellent), a self-reported subjective stress level of the user, a self-reported subjective fatigue level of the user, a self-reported subjective health status of the user, a recent life event experienced by the user, or any combination thereof.

The electronic interface 119 is configured to receive data (e.g., physiological data and/or audio data) from the one or more sensors 130 such that the data can be stored in the memory device 114 and/or analyzed by the processor 112 of the control system 110. The electronic interface 119 can communicate with the one or more sensors 130 using a wired connection or a wireless connection (e.g., using an RF communication protocol, a WiFi communication protocol, a Bluetooth communication protocol, over a cellular network, etc.). The electronic interface 119 can include an antenna, a receiver (e.g., an RF receiver), a transmitter (e.g., an RF transmitter), a transceiver, or any combination thereof. The electronic interface 119 can also include one more processors and/or one more memory devices that are the same as, or similar to, the processor 112 and the memory device 114 described herein. In some implementations, the electronic interface 119 is coupled to or integrated in the user device 170. In other implementations, the electronic interface 119 is coupled to or integrated (e.g., in a housing) with the control system 110 and/or the memory device 114.

As noted above, in some implementations, the system 100 optionally includes a respiratory therapy system 120 (also referred to as a respiratory system). The respiratory therapy system 120 can include a respiratory pressure therapy device 122 (referred to herein as respiratory therapy device 122, or flow generator), a user interface 124, a conduit 126 (also referred to as a tube or an air circuit), a display device 128, a humidification tank 129, or any combination thereof. In some implementations, the control system 110, the memory device 114, the display device 128, one or more of the sensors 130, and the humidification tank 129 are part of the respiratory therapy device 122. Respiratory pressure therapy refers to the application of a supply of air to an entrance to a user's airways at a controlled target pressure that is nominally positive with respect to atmosphere throughout the user's breathing cycle (e.g., in contrast to negative pressure therapies such as the tank ventilator or cuirass). The respiratory therapy system 120 is generally used to treat individuals suffering from one or more sleep-related respiratory disorders (e.g., obstructive sleep apnea, central sleep apnea, or mixed sleep apnea).

The respiratory therapy device 122 is generally used to generate pressurized air that is delivered to a user (e.g., using one or more motors that drive one or more compressors). In some implementations, the respiratory therapy device 122 generates continuous constant air pressure that is delivered to the user. In other implementations, the respiratory therapy device 122 generates two or more predetermined pressures (e.g., a first predetermined air pressure and a second predetermined air pressure). In still other implementations, the respiratory therapy device 122 is configured to generate a variety of different air pressures within a predetermined range. For example, the respiratory therapy device 122 can deliver at least about 6 cm H₂O, at least about 10 cm H₂O, at least about 20 cm H₂O, between about 6 cm H₂O and about 10 cm H₂O, between about 7 cm H₂O and about 12 cm H₂O, etc. The respiratory therapy device 122 can also deliver pressurized air at a predetermined flow rate between, for example, about -20 L/min and about 150 L/min, while maintaining a positive pressure (relative to the ambient pressure).

The user interface 124 engages a portion of the user's face and delivers pressurized air from the respiratory therapy device 122 to the user's airway to aid in preventing the airway from narrowing and/or collapsing during sleep. This may also increase the user's oxygen intake during sleep. Depending upon the therapy to be applied, the user interface 124 may form a seal, for example, with a region or portion of the user's face, to facilitate the delivery of gas at a pressure at sufficient variance with ambient pressure to effect therapy, for example, at a positive pressure of about 10 cm H₂O relative to ambient pressure. For other forms of therapy, such as the delivery of oxygen, the user interface may not include a seal sufficient to facilitate delivery to the airways of a supply of gas at a positive pressure of about 10 cm H₂O.

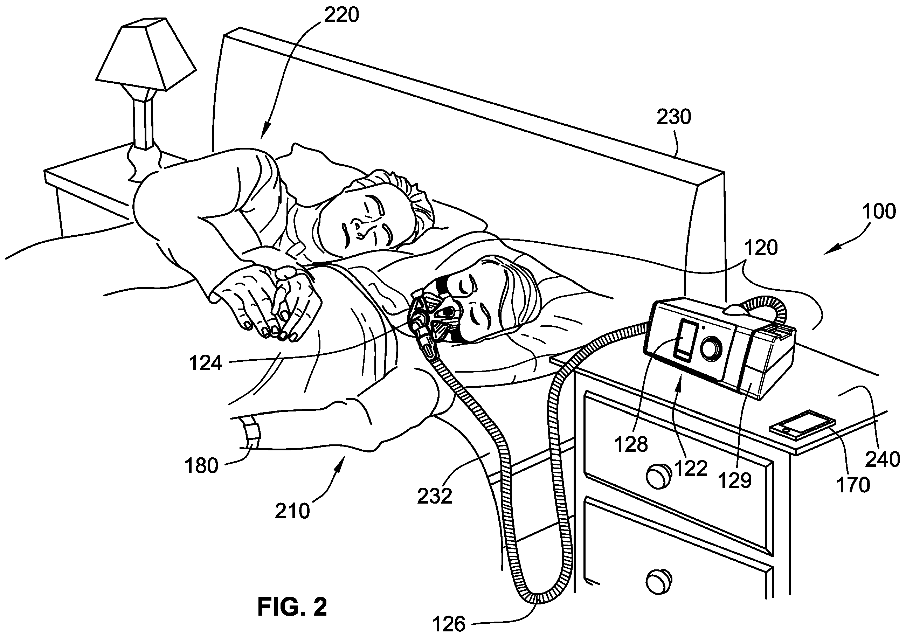

As shown in FIG. 2, in some implementations, the user interface 124 is a face mask that covers the nose and mouth of the user. Alternatively, the user interface 124 can be a nasal mask that provides air to the nose of the user or a nasal pillow mask that delivers air directly to the nostrils of the user. The user interface 124 can include a plurality of straps (e.g., including hook and loop fasteners) for positioning and/or stabilizing the interface on a portion of the user (e.g., the face) and a conformal cushion (e.g., silicone, plastic, foam, etc.) that aids in providing an air-tight seal between the user interface 124 and the user. The user interface 124 can also include one or more vents for permitting the escape of carbon dioxide and other gases exhaled by the user 210. In other implementations, the user interface 124 can include a mouthpiece (e.g., a night guard mouthpiece molded to conform to the user's teeth, a mandibular repositioning device, etc.).

The conduit 126 (also referred to as an air circuit or tube) allows the flow of air between two components of a respiratory therapy system 120, such as the respiratory therapy device 122 and the user interface 124. In some implementations, there can be separate limbs of the conduit for inhalation and exhalation. In other implementations, a single limb conduit is used for both inhalation and exhalation.

One or more of the respiratory therapy device 122, the user interface 124, the conduit 126, the display device 128, and the humidification tank 129 can contain one or more sensors (e.g., a pressure sensor, a flow rate sensor, or more generally any of the other sensors 130 described herein). These one or more sensors can be use, for example, to measure the air pressure and/or flow rate of pressurized air supplied by the respiratory therapy device 122.

The display device 128 is generally used to display image(s) including still images, video images, or both and/or information regarding the respiratory therapy device 122. For example, the display device 128 can provide information regarding the status of the respiratory therapy device 122 (e.g., whether the respiratory therapy device 122 is on/off, the pressure of the air being delivered by the respiratory therapy device 122, the temperature of the air being delivered by the respiratory therapy device 122, etc.) and/or other information (e.g., a sleep score or a therapy score (also referred to as a myAir^{™} score), the current date/time, personal information for the user 210, etc.). In some implementations, the display device 128 acts as a human-machine interface (HMI) that includes a graphic user interface (GUI) configured to display the image(s) as an input interface. The display device 128 can be an LED display, an OLED display, an LCD display, or the like. The input interface can be, for example, a touchscreen or touch-sensitive substrate, a mouse, a keyboard, or any sensor system configured to sense inputs made by a human user interacting with the respiratory therapy device 122.

The humidification tank 129 is coupled to or integrated in the respiratory therapy device 122 and includes a reservoir of water that can be used to humidify the pressurized air delivered from the respiratory therapy device 122. The respiratory therapy device 122 can include a heater to heat the water in the humidification tank 129 in order to humidify the pressurized air provided to the user. Additionally, in some implementations, the conduit 126 can also include a heating element (e.g., coupled to and/or imbedded in the conduit 126) that heats the pressurized air delivered to the user.

The respiratory therapy system 120 can be used, for example, as a ventilator or a positive airway pressure (PAP) system such as a continuous positive airway pressure (CPAP) system, an automatic positive airway pressure system (APAP), a bi-level or variable positive airway pressure system (BPAP or VPAP), or any combination thereof. The CPAP system delivers a predetermined air pressure (e.g., determined by a sleep physician) to the user. The APAP system automatically varies the air pressure delivered to the user based on, for example, respiration data associated with the user. The BPAP or VPAP system is configured to deliver a first predetermined pressure (e.g., an inspiratory positive airway pressure or IPAP) and a second predetermined pressure (e.g., an expiratory positive airway pressure or EPAP) that is lower than the first predetermined pressure.

Referring to FIG. 2, a portion of the system 100 (FIG. 1), according to some implementations, is illustrated. A user 210 of the respiratory therapy system 120 and a bed partner 220 are located in a bed 230 and are laying on a mattress 232. The user interface 124 (e.g., a full face mask) can be worn by the user 210 during a sleep session. The user interface 124 is fluidly coupled and/or connected to the respiratory therapy device 122 via the conduit 126. In turn, the respiratory therapy device 122 delivers pressurized air to the user 210 via the conduit 126 and the user interface 124 to increase the air pressure in the throat of the user 210 to aid in preventing the airway from closing and/or narrowing during sleep. The respiratory therapy device 122 can be positioned on a nightstand 240 that is directly adjacent to the bed 230 as shown in FIG. 2, or more generally, on any surface or structure that is generally adjacent to the bed 230 and/or the user 210.

Referring to back to FIG. 1, the one or more sensors 130 of the system 100 include a pressure sensor 132, a flow rate sensor 134, temperature sensor 136, a motion sensor 138, a microphone 140, a speaker 142, a radio-frequency (RF) receiver 146, a RF transmitter 148, a camera 150, an infrared sensor 152, a photoplethysmogram (PPG) sensor 154, an electrocardiogram (ECG) sensor 156, an electroencephalography (EEG) sensor 158, a capacitive sensor 160, a force sensor 162, a strain gauge sensor 164, an electromyography (EMG) sensor 166, an oxygen sensor 168, an analyte sensor 174, a moisture sensor 176, a LiDAR sensor 178, or any combination thereof. Generally, each of the one or more sensors 130 are configured to output sensor data that is received and stored in the memory device 114 or one or more other memory devices.

While the one or more sensors 130 are shown and described as including each of the pressure sensor 132, the flow rate sensor 134, the temperature sensor 136, the motion sensor 138, the microphone 140, the speaker 142, the RF receiver 146, the RF transmitter 148, the camera 150, the infrared sensor 152, the photoplethysmogram (PPG) sensor 154, the electrocardiogram (ECG) sensor 156, the electroencephalography (EEG) sensor 158, the capacitive sensor 160, the force sensor 162, the strain gauge sensor 164, the electromyography (EMG) sensor 166, the oxygen sensor 168, the analyte sensor 174, the moisture sensor 176, and the LiDAR sensor 178, more generally, the one or more sensors 130 can include any combination and any number of each of the sensors described and/or shown herein.

The one or more sensors 130 can be used to generate, for example, physiological data, audio data, or both. Physiological data generated by one or more of the sensors 130 can be used by the control system 110 to determine a sleep-wake signal associated with a user during a sleep session and one or more sleep-related parameters. The sleep-wake signal can be indicative of one or more sleep states, including wakefulness, relaxed wakefulness, micro-awakenings, a rapid eye movement (REM) stage, a first non-REM stage (often referred to as "N1"), a second non-REM stage (often referred to as "N2"), a third non-REM stage (often referred to as "N3"), or any combination thereof. The sleep-wake signal can also be timestamped to indicate a time that the user enters the bed, a time that the user exits the bed, a time that the user attempts to fall asleep, etc. The sleep-wake signal can be measured by the sensor(s) 130 during the sleep session at a predetermined sampling rate, such as, for example, one sample per second, one sample per 30 seconds, one sample per minute, etc. Examples of the one or more sleep-related parameters that can be determined for the user during the sleep session based on the sleep-wake signal include a total time in bed, a total sleep time, a sleep onset latency, a wake-after-sleep-onset parameter, a sleep efficiency, a fragmentation index, or any combination thereof. Throughout a typical sleep session, an individual moves between the four sleep stages, in patterns that create distinct sleep cycles. A full sleep cycle typically lasts between 90-100 minutes. Over the course of a full sleep sessions, the individual will generally complete 4-5 sleep cycles. Sleep stages, and sleep cycles, can be depicted in a hypnogram, as further described herein.

Physiological data and/or audio data generated by the one or more sensors 130 can also be used to determine a respiration signal associated with a user during a sleep session. The respiration signal is generally indicative of respiration or breathing of the user during the sleep session. The respiration signal can be indicative of, for example, a respiration rate, a respiration rate variability, an inspiration amplitude, an expiration amplitude, an inspiration-expiration ratio, a number of events per hour, a pattern of events, pressure settings of the respiratory therapy device 122, or any combination thereof. The event(s) can include snoring, apneas, central apneas, obstructive apneas, mixed apneas, hypopneas, a mask leak (e.g., from the user interface 124), a restless leg, a sleeping disorder, choking, an increased heart rate, labored breathing, an asthma attack, an epileptic episode, a seizure, or any combination thereof.

The pressure sensor 132 outputs pressure data that can be stored in the memory device 114 and/or analyzed by the processor 112 of the control system 110. In some implementations, the pressure sensor 132 is an air pressure sensor (e.g., barometric pressure sensor) that generates sensor data indicative of the respiration (e.g., inhaling and/or exhaling) of the user of the respiratory therapy system 120 and/or ambient pressure. In such implementations, the pressure sensor 132 can be coupled to or integrated in the respiratory therapy device 122. The pressure sensor 132 can be, for example, a capacitive sensor, an electromagnetic sensor, a piezoelectric sensor, a strain-gauge sensor, an optical sensor, a potentiometric sensor, or any combination thereof. In one example, the pressure sensor 132 can be used to determine a blood pressure of a user.

The flow rate sensor 134 outputs flow rate data that can be stored in the memory device 114 and/or analyzed by the processor 112 of the control system 110. In some implementations, the flow rate sensor 134 is used to determine an air flow rate from the respiratory therapy device 122, an air flow rate through the conduit 126, an air flow rate through the user interface 124, or any combination thereof. In such implementations, the flow rate sensor 134 can be coupled to or integrated in the respiratory therapy device 122, the user interface 124, or the conduit 126. The flow rate sensor 134 can be a mass flow rate sensor such as, for example, a rotary flow meter (e.g., Hall effect flow meters), a turbine flow meter, an orifice flow meter, an ultrasonic flow meter, a hot wire sensor, a vortex sensor, a membrane sensor, or any combination thereof.

The temperature sensor 136 outputs temperature data that can be stored in the memory device 114 and/or analyzed by the processor 112 of the control system 110. In some implementations, the temperature sensor 136 generates temperatures data indicative of a core body temperature of the user 210 (FIG. 2), a skin temperature of the user 210, a temperature of the air flowing from the respiratory therapy device 122 and/or through the conduit 126, a temperature in the user interface 124, an ambient temperature, or any combination thereof. The temperature sensor 136 can be, for example, a thermocouple sensor, a thermistor sensor, a silicon band gap temperature sensor or semiconductor-based sensor, a resistance temperature detector, or any combination thereof.

The microphone 140 outputs audio data that can be stored in the memory device 114 and/or analyzed by the processor 112 of the control system 110. The audio data generated by the microphone 140 is reproducible as one or more sound(s) during a sleep session (e.g., sounds from the user 210). The audio data from the microphone 140 can also be used to identify (e.g., using the control system 110) an event experienced by the user during the sleep session, as described in further detail herein. The microphone 140 can be coupled to or integrated in the respiratory therapy device 122, the use interface 124, the conduit 126, or the user device 170.

The speaker 142 outputs sound waves that are typically audible to a user of the system 100 (e.g., the user 210 of FIG. 2). The speaker 142 can be used, for example, as an alarm clock or to play an alert or message to the user 210 (e.g., in response to an event). In some implementations, the speaker 142 can be used to communicate the audio data generated by the microphone 140 to the user. The speaker 142 can be coupled to or integrated in the respiratory therapy device 122, the user interface 124, the conduit 126, or the user device 170.

The microphone 140 and the speaker 142 can be used as separate devices. In some implementations, the microphone 140 and the speaker 142 can be combined into an acoustic sensor 141, as described in, for example, WO 2018/050913 and WO 2020/104465, each of which is hereby incorporated by reference herein in its entirety. In such implementations, the speaker 142 generates or emits sound waves at a predetermined interval and the microphone 140 detects the reflections of the emitted sound waves from the speaker 142. The sound waves generated or emitted by the speaker 142 have a frequency that is not audible to the human ear (e.g., below 20 Hz or above around 18 kHz) so as not to disturb the sleep of the user 210 or the bed partner 220 (FIG. 2). Based at least in part on the data from the microphone 140 and/or the speaker 142, the control system 110 can determine a location of the user 210 (FIG. 2) and/or one or more of the sleep-related parameters described in herein.

In some implementations, the sensors 130 include (i) a first microphone that is the same as, or similar to, the microphone 140, and is integrated in the acoustic sensor 141 and (ii) a second microphone that is the same as, or similar to, the microphone 140, but is separate and distinct from the first microphone that is integrated in the acoustic sensor 141.

The RF transmitter 148 generates and/or emits radio waves having a predetermined frequency and/or a predetermined amplitude (e.g., within a high frequency band, within a low frequency band, long wave signals, short wave signals, etc.). The RF receiver 146 detects the reflections of the radio waves emitted from the RF transmitter 148, and this data can be analyzed by the control system 110 to determine a location of the user 210 (FIG. 2) and/or one or more of the sleep-related parameters described herein. An RF receiver (either the RF receiver 146 and the RF transmitter 148 or another RF pair) can also be used for wireless communication between the control system 110, the respiratory therapy device 122, the one or more sensors 130, the user device 170, or any combination thereof. While the RF receiver 146 and RF transmitter 148 are shown as being separate and distinct elements in FIG. 1, in some implementations, the RF receiver 146 and RF transmitter 148 are combined as a part of an RF sensor 147. In some such implementations, the RF sensor 147 includes a control circuit. The specific format of the RF communication can be WiFi, Bluetooth, or the like.

In some implementations, the RF sensor 147 is a part of a mesh system. One example of a mesh system is a WiFi mesh system, which can include mesh nodes, mesh router(s), and mesh gateway(s), each of which can be mobile/movable or fixed. In such implementations, the WiFi mesh system includes a WiFi router and/or a WiFi controller and one or more satellites (e.g., access points), each of which include an RF sensor that the is the same as, or similar to, the RF sensor 147. The WiFi router and satellites continuously communicate with one another using WiFi signals. The WiFi mesh system can be used to generate motion data based on changes in the WiFi signals (e.g., differences in received signal strength) between the router and the satellite(s) due to an object or person moving partially obstructing the signals. The motion data can be indicative of motion, breathing, heart rate, gait, falls, behavior, etc., or any combination thereof.

The camera 150 outputs image data reproducible as one or more images (e.g., still images, video images, thermal images, or a combination thereof) that can be stored in the memory device 114. The image data from the camera 150 can be used by the control system 110 to determine one or more of the sleep-related parameters described herein. For example, the image data from the camera 150 can be used to identify a location of the user, to determine a time when the user 210 enters the bed 230 (FIG. 2), and to determine a time when the user 210 exits the bed 230.

The infrared (IR) sensor 152 outputs infrared image data reproducible as one or more infrared images (e.g., still images, video images, or both) that can be stored in the memory device 114. The infrared data from the IR sensor 152 can be used to determine one or more sleep-related parameters during a sleep session, including a temperature of the user 210 and/or movement of the user 210. The IR sensor 152 can also be used in conjunction with the camera 150 when measuring the presence, location, and/or movement of the user 210. The IR sensor 152 can detect infrared light having a wavelength between about 700 nm and about 1 mm, for example, while the camera 150 can detect visible light having a wavelength between about 380 nm and about 740 nm.

The PPG sensor 154 outputs physiological data associated with the user 210 (FIG. 2) that can be used to determine one or more sleep-related parameters, such as, for example, a heart rate, a heart rate variability, a cardiac cycle, respiration rate, an inspiration amplitude, an expiration amplitude, an inspiration-expiration ratio, estimated blood pressure parameter(s), or any combination thereof. The PPG sensor 154 can be worn by the user 210, embedded in clothing and/or fabric that is worn by the user 210, embedded in and/or coupled to the user interface 124 and/or its associated headgear (e.g., straps, etc.), etc.

The ECG sensor 156 outputs physiological data associated with electrical activity of the heart of the user 210. In some implementations, the ECG sensor 156 includes one or more electrodes that are positioned on or around a portion of the user 210 during the sleep session. The physiological data from the ECG sensor 156 can be used, for example, to determine one or more of the sleep-related parameters described herein.

The EEG sensor 158 outputs physiological data associated with electrical activity of the brain of the user 210. In some implementations, the EEG sensor 158 includes one or more electrodes that are positioned on or around the scalp of the user 210 during the sleep session. The physiological data from the EEG sensor 158 can be used, for example, to determine a sleep state of the user 210 at any given time during the sleep session. In some implementations, the EEG sensor 158 can be integrated in the user interface 124 and/or the associated headgear (e.g., straps, etc.).

The capacitive sensor 160, the force sensor 162, and the strain gauge sensor 164 output data that can be stored in the memory device 114 and used by the control system 110 to determine one or more of the sleep-related parameters described herein. The EMG sensor 166 outputs physiological data associated with electrical activity produced by one or more muscles. The oxygen sensor 168 outputs oxygen data indicative of an oxygen concentration of gas (e.g., in the conduit 126 or at the user interface 124). The oxygen sensor 168 can be, for example, an ultrasonic oxygen sensor, an electrical oxygen sensor, a chemical oxygen sensor, an optical oxygen sensor, or any combination thereof. In some implementations, the one or more sensors 130 also include a galvanic skin response (GSR) sensor, a blood flow sensor, a respiration sensor, a pulse sensor, a sphygmomanometer sensor, an oximetry sensor, or any combination thereof.

The analyte sensor 174 can be used to detect the presence of an analyte in the exhaled breath of the user 210. The data output by the analyte sensor 174 can be stored in the memory device 114 and used by the control system 110 to determine the identity and concentration of any analytes in the breath of the user 210. In some implementations, the analyte sensor 174 is positioned near a mouth of the user 210 to detect analytes in breath exhaled from the user 210's mouth. For example, when the user interface 124 is a face mask that covers the nose and mouth of the user 210, the analyte sensor 174 can be positioned within the face mask to monitor the user 210's mouth breathing. In other implementations, such as when the user interface 124 is a nasal mask or a nasal pillow mask, the analyte sensor 174 can be positioned near the nose of the user 210 to detect analytes in breath exhaled through the user's nose. In still other implementations, the analyte sensor 174 can be positioned near the user 210's mouth when the user interface 124 is a nasal mask or a nasal pillow mask. In this implementation, the analyte sensor 174 can be used to detect whether any air is inadvertently leaking from the user 210's mouth. In some implementations, the analyte sensor 174 is a volatile organic compound (VOC) sensor that can be used to detect carbon-based chemicals or compounds. In some implementations, the analyte sensor 174 can also be used to detect whether the user 210 is breathing through their nose or mouth. For example, if the data output by an analyte sensor 174 positioned near the mouth of the user 210 or within the face mask (in implementations where the user interface 124 is a face mask) detects the presence of an analyte, the control system 110 can use this data as an indication that the user 210 is breathing through their mouth.

The moisture sensor 176 outputs data that can be stored in the memory device 114 and used by the control system 110. The moisture sensor 176 can be used to detect moisture in various areas surrounding the user (e.g., inside the conduit 126 or the user interface 124, near the user 210's face, near the connection between the conduit 126 and the user interface 124, near the connection between the conduit 126 and the respiratory therapy device 122, etc.). Thus, in some implementations, the moisture sensor 176 can be coupled to or integrated in the user interface 124 or in the conduit 126 to monitor the humidity of the pressurized air from the respiratory therapy device 122. In other implementations, the moisture sensor 176 is placed near any area where moisture levels need to be monitored. The moisture sensor 176 can also be used to monitor the humidity of the ambient environment surrounding the user 210, for example, the air inside the bedroom.

The Light Detection and Ranging (LiDAR) sensor 178 can be used for depth sensing. This type of optical sensor (e.g., laser sensor) can be used to detect objects and build three dimensional (3D) maps of the surroundings, such as of a living space. LiDAR can generally utilize a pulsed laser to make time of flight measurements. LiDAR is also referred to as 3D laser scanning. In an example of use of such a sensor, a fixed or mobile device (such as a smartphone) having a LiDAR sensor 166 can measure and map an area extending 5 meters or more away from the sensor. The LiDAR data can be fused with point cloud data estimated by an electromagnetic RADAR sensor, for example. The LiDAR sensor(s) 178 can also use artificial intelligence (AI) to automatically geofence RADAR systems by detecting and classifying features in a space that might cause issues for RADAR systems, such a glass windows (which can be highly reflective to RADAR). LiDAR can also be used to provide an estimate of the height of a person, as well as changes in height when the person sits down, or falls down, for example. LiDAR may be used to form a 3D mesh representation of an environment. In a further use, for solid surfaces through which radio waves pass (e.g., radio-translucent materials), the LiDAR may reflect off such surfaces, thus allowing a classification of different type of obstacles.

While shown separately in FIG. 1, any combination of the one or more sensors 130 can be integrated in and/or coupled to any one or more of the components of the system 100, including the respiratory therapy device 122, the user interface 124, the conduit 126, the humidification tank 129, the control system 110, the user device 170 (e.g., a smart device such as a smartphone), or any combination thereof. For example, the microphone 140 and/or speaker 142 is integrated in and/or coupled to the user device 170 and the pressure sensor 130 and/or flow rate sensor 132 are integrated in and/or coupled to the respiratory therapy device 122. In some implementations, at least one of the one or more sensors 130 is not coupled to the respiratory therapy device 122, the control system 110, or the user device 170, and is positioned generally adjacent to the user 210 during the sleep session (e.g., positioned on or in contact with a portion of the user 210, worn by the user 210, coupled to or positioned on the nightstand, coupled to the mattress, coupled to the ceiling, etc.).

The user device 170 (FIG. 1) includes a display device 172. The user device 170 can be, for example, a mobile device such as a smart phone, a tablet, a laptop, or the like. Alternatively, the user device 170 can be an external sensing system, a television (e.g., a smart television) or another smart home device (e.g., a smart speaker(s) such as Google Home^{™}, Google Nest^{™}, Amazon Echo^{™}, Amazon Echo Show^{™}, Alexa^{™}-enabled devices, etc.). In some implementations, the user device is a wearable device (e.g., a smart watch). The display device 172 is generally used to display image(s) including still images, video images, or both. In some implementations, the display device 172 acts as a human-machine interface (HMI) that includes a graphic user interface (GUI) configured to display the image(s) and an input interface. The display device 172 can be an LED display, an OLED display, an LCD display, or the like. The input interface can be, for example, a touchscreen or touch-sensitive substrate, a mouse, a keyboard, or any sensor system configured to sense inputs made by a human user interacting with the user device 170. In some implementations, one or more user devices can be used by and/or included in the system 100.

The activity tracker 180 is generally used to aid in generating physiological data for determining an activity measurement associated with the user. The activity measurement can include, for example, a number of steps, a distance traveled, a number of steps climbed, a duration of physical activity, a type of physical activity, an intensity of physical activity, time spent standing, a respiration rate, an average respiration rate, a resting respiration rate, a maximum respiration rate, a respiration rate variability, a heart rate, an average heart rate, a resting heart rate, a maximum heart rate, a heart rate variability, a number of calories burned, blood oxygen saturation, electrodermal activity (also known as skin conductance or galvanic skin response), a position of the user, a posture of the user, or any combination thereof. The activity tracker 180 includes one or more of the sensors 130 described herein, such as, for example, the motion sensor 138 (e.g., one or more accelerometers and/or gyroscopes), the PPG sensor 154, and/or the ECG sensor 156.

In some implementations, the activity tracker 180 is a wearable device that can be worn by the user, such as a smartwatch, a wristband, a ring, or a patch. For example, referring to FIG. 2, the activity tracker 180 is worn on a wrist of the user 210. The activity tracker 180 can also be coupled to or integrated a garment or clothing that is worn by the user. Alternatively still, the activity tracker 180 can also be coupled to or integrated in (e.g., within the same housing) the user device 170. More generally, the activity tracker 180 can be communicatively coupled with, or physically integrated in (e.g., within a housing), the control system 110, the memory 114, the respiratory therapy system 120, and/or the user device 170.

While the control system 110 and the memory device 114 are described and shown in FIG. 1 as being a separate and distinct component of the system 100, in some implementations, the control system 110 and/or the memory device 114 are integrated in the user device 170 and/or the respiratory therapy device 122. Alternatively, in some implementations, the control system 110 or a portion thereof (e.g., the processor 112) can be located in a cloud (e.g., integrated in a server, integrated in an Internet of Things (IoT) device, connected to the cloud, be subject to edge cloud processing, etc.), located in one or more servers (e.g., remote servers, local servers, etc., or any combination thereof.

While system 100 is shown as including all of the components described above, more or fewer components can be included in a system for generating physiological data and determining a recommended notification or action for the user according to implementations of the present disclosure. For example, a first alternative system includes the control system 110, the memory device 114, and at least one of the one or more sensors 130. As another example, a second alternative system includes the control system 110, the memory device 114, at least one of the one or more sensors 130, and the user device 170. As yet another example, a third alternative system includes the control system 110, the memory device 114, the respiratory therapy system 120, at least one of the one or more sensors 130, and the user device 170. Thus, various systems can be formed using any portion or portions of the components shown and described herein and/or in combination with one or more other components.

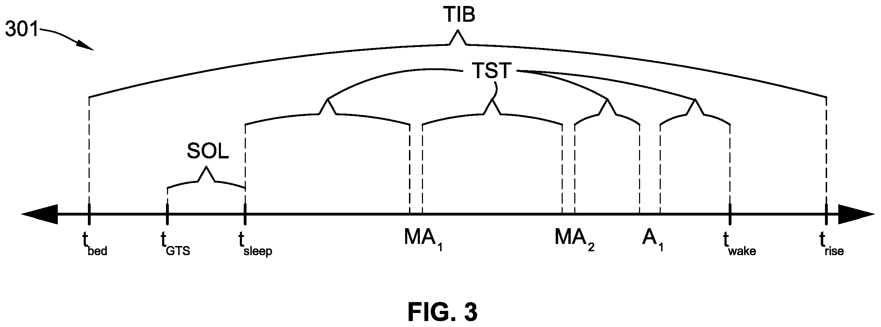

As used herein, a sleep session can be defined in a number of ways based on, for example, an initial start time and an end time. Referring to FIG. 3, an exemplary timeline 300 for a sleep session is illustrated. The timeline 300 includes an enter bed time (t_{bed}), a go-to-sleep time (t_{GTS}), an initial sleep time (tₛₗₑₑₚ), a first micro-awakening MA₁ and a second micro-awakening MA₂, a wake-up time (t_{wake}), and a rising time (tᵣᵢₛₑ).

As used herein, a sleep session can be defined in multiple ways. For example, a sleep session can be defined by an initial start time and an end time. In some implementations, a sleep session is a duration where the user is asleep, that is, the sleep session has a start time and an end time, and during the sleep session, the user does not wake until the end time. That is, any period of the user being awake is not included in a sleep session. From this first definition of sleep session, if the user wakes ups and falls asleep multiple times in the same night, each of the sleep intervals separated by an awake interval is a sleep session.

Alternatively, in some implementations, a sleep session has a start time and an end time, and during the sleep session, the user can wake up, without the sleep session ending, so long as a continuous duration that the user is awake is below an awake duration threshold. The awake duration threshold can be defined as a percentage of a sleep session. The awake duration threshold can be, for example, about twenty percent of the sleep session, about fifteen percent of the sleep session duration, about ten percent of the sleep session duration, about five percent of the sleep session duration, about two percent of the sleep session duration, etc., or any other threshold percentage. In some implementations, the awake duration threshold is defined as a fixed amount of time, such as, for example, about one hour, about thirty minutes, about fifteen minutes, about ten minutes, about five minutes, about two minutes, etc., or any other amount of time.

In some implementations, a sleep session is defined as the entire time between the time in the evening at which the user first entered the bed, and the time the next morning when user last left the bed. Put another way, a sleep session can be defined as a period of time that begins on a first date (e.g., Monday, January 6, 2020) at a first time (e.g., 10:00 PM), that can be referred to as the current evening, when the user first enters a bed with the intention of going to sleep (e.g., not if the user intends to first watch television or play with a smart phone before going to sleep, etc.), and ends on a second date (e.g., Tuesday, January 7, 2020) at a second time (e.g., 7:00 AM), that can be referred to as the next morning, when the user first exits the bed with the intention of not going back to sleep that next morning.

In some implementations, the user can manually define the beginning of a sleep session and/or manually terminate a sleep session. For example, the user can select (e.g., by clicking or tapping) one or more user-selectable element that is displayed on the display device 172 of the user device 170 (FIG. 1) to manually initiate or terminate the sleep session.

Referring to FIG. 3, an exemplary timeline 300 for a sleep session is illustrated. The timeline 300 includes an enter bed time (t_{bed}), a go-to-sleep time (t_{GTS}), an initial sleep time (tₛₗₑₑₚ), a first micro-awakening MA₁, a second micro-awakening MA₂, an awakening A, a wake-up time (t_{wake}), and a rising time (tᵣᵢₛₑ).

The enter bed time t_{bed} is associated with the time that the user initially enters the bed (e.g., bed 230 in FIG. 2) prior to falling asleep (e.g., when the user lies down or sits in the bed). The enter bed time t_{bed} can be identified based on a bed threshold duration to distinguish between times when the user enters the bed for sleep and when the user enters the bed for other reasons (e.g., to watch TV). For example, the bed threshold duration can be at least about 10 minutes, at least about 20 minutes, at least about 30 minutes, at least about 45 minutes, at least about 1 hour, at least about 2 hours, etc. While the enter bed time t_{bed} is described herein in reference to a bed, more generally, the enter time t_{bed} can refer to the time the user initially enters any location for sleeping (e.g., a couch, a chair, a sleeping bag, etc.).

The go-to-sleep time (GTS) is associated with the time that the user initially attempts to fall asleep after entering the bed (t_{bed}). For example, after entering the bed, the user may engage in one or more activities to wind down prior to trying to sleep (e.g., reading, watching TV, listening to music, using the user device 170, etc.). The initial sleep time (tₛₗₑₑₚ) is the time that the user initially falls asleep. For example, the initial sleep time (tₛₗₑₑₚ) can be the time that the user initially enters the first non-REM sleep stage.

The wake-up time t_{wake} is the time associated with the time when the user wakes up without going back to sleep (e.g., as opposed to the user waking up in the middle of the night and going back to sleep). The user may experience one of more unconscious microawakenings (e.g., microawakenings MA₁ and MA₂) having a short duration (e.g., 5 seconds, 10 seconds, 30 seconds, 1 minute, etc.) after initially falling asleep. In contrast to the wake-up time t_{wake}, the user goes back to sleep after each of the microawakenings MA₁ and MA₂. Similarly, the user may have one or more conscious awakenings (e.g., awakening A) after initially falling asleep (e.g., getting up to go to the bathroom, attending to children or pets, sleep walking, etc.). However, the user goes back to sleep after the awakening A. Thus, the wake-up time t_{wake} can be defined, for example, based on a wake threshold duration (e.g., the user is awake for at least 15 minutes, at least 20 minutes, at least 30 minutes, at least 1 hour, etc.).

Similarly, the rising time tᵣᵢₛₑ is associated with the time when the user exits the bed and stays out of the bed with the intent to end the sleep session (e.g., as opposed to the user getting up during the night to go to the bathroom, to attend to children or pets, sleep walking, etc.). In other words, the rising time tᵣᵢₛₑ is the time when the user last leaves the bed without returning to the bed until a next sleep session (e.g., the following evening). Thus, the rising time tᵣᵢₛₑ can be defined, for example, based on a rise threshold duration (e.g., the user has left the bed for at least 15 minutes, at least 20 minutes, at least 30 minutes, at least 1 hour, etc.). The enter bed time t_{bed} time for a second, subsequent sleep session can also be defined based on a rise threshold duration (e.g., the user has left the bed for at least 4 hours, at least 6 hours, at least 8 hours, at least 12 hours, etc.).

As described above, the user may wake up and get out of bed one more times during the night between the initial t_{bed} and the final tᵣᵢₛₑ. In some implementations, the final wake-up time t_{wake} and/or the final rising time tᵣᵢₛₑ that are identified or determined based on a predetermined threshold duration of time subsequent to an event (e.g., falling asleep or leaving the bed). Such a threshold duration can be customized for the user. For a standard user which goes to bed in the evening, then wakes up and goes out of bed in the morning any period (between the user waking up (t_{wake}) or raising up (tᵣᵢₛₑ), and the user either going to bed (t_{bed}), going to sleep (T_{GTS}) or falling asleep (tₛₗₑₑₚ) of between about 12 and about 18 hours can be used. For users that spend longer periods of time in bed, shorter threshold periods may be used (e.g., between about 8 hours and about 14 hours). The threshold period may be initially selected and/or later adjusted based on the system monitoring the user's sleep behavior.

The total time in bed (TIB) is the duration of time between the time enter bed time t_{bed} and the rising time tᵣᵢₛₑ. The total sleep time (TST) is associated with the duration between the initial sleep time and the wake-up time, excluding any conscious or unconscious awakenings and/or micro-awakenings therebetween. Generally, the total sleep time (TST) will be shorter than the total time in bed (TIB) (e.g., one minute short, ten minutes shorter, one hour shorter, etc.). For example, referring to the timeline 300 of FIG. 3, the total sleep time (TST) spans between the initial sleep time tₛₗₑₑₚ and the wake-up time t_{wake}, but excludes the duration of the first micro-awakening MA₁, the second micro-awakening MA₂, and the awakening A. As shown, in this example, the total sleep time (TST) is shorter than the total time in bed (TIB).

In some implementations, the total sleep time (TST) can be defined as a persistent total sleep time (PTST). In such implementations, the persistent total sleep time excludes a predetermined initial portion or period of the first non-REM stage (e.g., light sleep stage). For example, the predetermined initial portion can be between about 30 seconds and about 20 minutes, between about 1 minute and about 10 minutes, between about 3 minutes and about 5 minutes, etc. The persistent total sleep time is a measure of sustained sleep, and smooths the sleep-wake hypnogram. For example, when the user is initially falling asleep, the user may be in the first non-REM stage for a very short time (e.g., about 30 seconds), then back into the wakefulness stage for a short period (e.g., one minute), and then goes back to the first non-REM stage. In this example, the persistent total sleep time excludes the first instance (e.g., about 30 seconds) of the first non-REM stage.

In some implementations, the sleep session is defined as starting at the enter bed time (t_{bed}) and ending at the rising time (tᵣᵢₛₑ), i.e., the sleep session is defined as the total time in bed (TIB). In some implementations, a sleep session is defined as starting at the initial sleep time (tₛₗₑₑₚ) and ending at the wake-up time (t_{wake}). In some implementations, the sleep session is defined as the total sleep time (TST). In some implementations, a sleep session is defined as starting at the go-to-sleep time (t_{GTS}) and ending at the wake-up time (t_{wake}). In some implementations, a sleep session is defined as starting at the go-to-sleep time (t_{GTS}) and ending at the rising time (tᵣᵢₛₑ). In some implementations, a sleep session is defined as starting at the enter bed time (t_{bed}) and ending at the wake-up time (t_{wake}). In some implementations, a sleep session is defined as starting at the initial sleep time (tₛₗₑₑₚ) and ending at the rising time (tᵣᵢₛₑ).

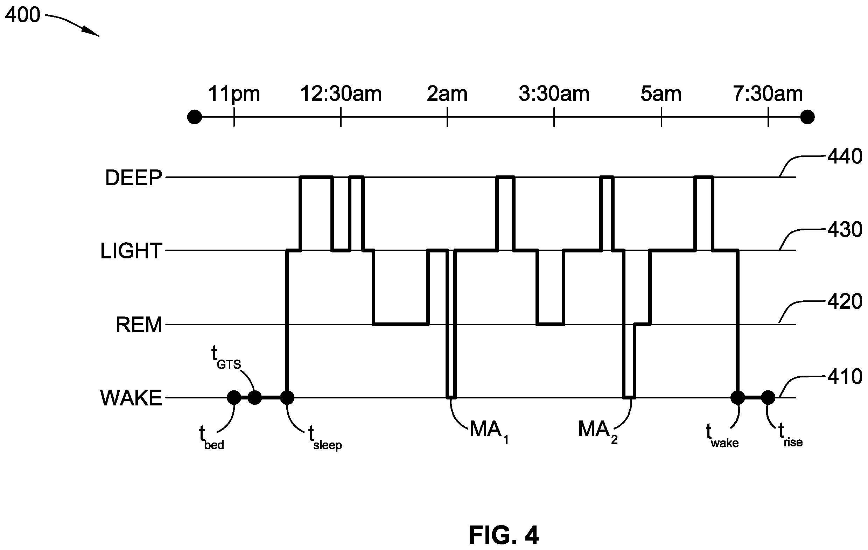

Referring to FIG. 4, an exemplary hypnogram 400 corresponding to the timeline 300 (FIG. 3), according to some implementations, is illustrated. As shown, the hypnogram 400 includes a sleep-wake signal 401, a wakefulness stage axis 410, a REM stage axis 420, a light sleep stage axis 430, and a deep sleep stage axis 440. The intersection between the sleep-wake signal 401 and one of the axes 410-440 is indicative of the sleep stage at any given time during the sleep session.

The sleep-wake signal 401 can be generated based on physiological data associated with the user (e.g., generated by one or more of the sensors 130 described herein). The sleep-wake signal can be indicative of one or more sleep states, including wakefulness, relaxed wakefulness, microawakenings, a REM stage, a first non-REM stage, a second non-REM stage, a third non-REM stage, or any combination thereof. In some implementations, one or more of the first non-REM stage, the second non-REM stage, and the third non-REM stage can be grouped together and categorized as a light sleep stage or a deep sleep stage. For example, the light sleep stage can include the first non-REM stage and the deep sleep stage can include the second non-REM stage and the third non-REM stage. While the hypnogram 400 is shown in FIG. 4 as including the light sleep stage axis 430 and the deep sleep stage axis 440, in some implementations, the hypnogram 400 can include an axis for each of the first non-REM stage, the second non-REM stage, and the third non-REM stage. In other implementations, the sleep-wake signal can also be indicative of a respiration signal, a respiration rate, an inspiration amplitude, an expiration amplitude, an inspiration-expiration ratio, a number of events per hour, a pattern of events, or any combination thereof. Information describing the sleep-wake signal can be stored in the memory device 114.

The hypnogram 400 can be used to determine one or more sleep-related parameters, such as, for example, a sleep onset latency (SOL), wake-after-sleep onset (WASO), a sleep efficiency (SE), a sleep fragmentation index, sleep blocks, or any combination thereof.

The sleep onset latency (SOL) is defined as the time between the go-to-sleep time (T_{GTS}) and the initial sleep time (tₛₗₑₑₚ). In other words, the sleep onset latency is indicative of the time that it took the user to actually fall asleep after initially attempting to fall asleep. In some implementations, the sleep onset latency is defined as a persistent sleep onset latency (PSOL). The persistent sleep onset latency differs from the sleep onset latency in that the persistent sleep onset latency is defined as the duration time between the go-to-sleep time and a predetermined amount of sustained sleep. In some implementations, the predetermined amount of sustained sleep can include, for example, at least 10 minutes of sleep within the second non-REM stage, the third non-REM stage, and/or the REM stage with no more than 2 minutes of wakefulness, the first non-REM stage, and/or movement therebetween. In other words, the persistent sleep onset latency requires up to, for example, 8 minutes of sustained sleep within the second non-REM stage, the third non-REM stage, and/or the REM stage. In other implementations, the predetermined amount of sustained sleep can include at least 10 minutes of sleep within the first non-REM stage, the second non-REM stage, the third non-REM stage, and/or the REM stage subsequent to the initial sleep time. In such implementations, the predetermined amount of sustained sleep can exclude any micro-awakenings (e.g., a ten second micro-awakening does not restart the 10-minute period).

The wake-after-sleep onset (WASO) is associated with the total duration of time that the user is awake between the initial sleep time and the wake-up time. Thus, the wake-after-sleep onset includes short and micro-awakenings during the sleep session (e.g., the micro-awakenings MA₁ and MA₂ shown in FIG. 4), whether conscious or unconscious. In some implementations, the wake-after-sleep onset (WASO) is defined as a persistent wake-after-sleep onset (PWASO) that only includes the total durations of awakenings having a predetermined length (e.g., greater than 10 seconds, greater than 30 seconds, greater than 60 seconds, greater than about 5 minutes, greater than about 10 minutes, etc.)

The sleep efficiency (SE) is determined as a ratio of the total time in bed (TIB) and the total sleep time (TST). For example, if the total time in bed is 8 hours and the total sleep time is 7.5 hours, the sleep efficiency for that sleep session is 93.75%. The sleep efficiency is indicative of the sleep hygiene of the user. For example, if the user enters the bed and spends time engaged in other activities (e.g., watching TV) before sleep, the sleep efficiency will be reduced (e.g., the user is penalized). In some implementations, the sleep efficiency (SE) can be calculated based on the total time in bed (TIB) and the total time that the user is attempting to sleep. In such implementations, the total time that the user is attempting to sleep is defined as the duration between the go-to-sleep (GTS) time and the rising time described herein. For example, if the total sleep time is 8 hours (e.g., between 11 PM and 7 AM), the go-to-sleep time is 10:45 PM, and the rising time is 7:15 AM, in such implementations, the sleep efficiency parameter is calculated as about 94%.

The fragmentation index is determined based at least in part on the number of awakenings during the sleep session. For example, if the user had two micro-awakenings (e.g., micro-awakening MA₁ and micro-awakening MA₂ shown in FIG. 4), the fragmentation index can be expressed as 2. In some implementations, the fragmentation index is scaled between a predetermined range of integers (e.g., between 0 and 10).

The sleep blocks are associated with a transition between any stage of sleep (e.g., the first non-REM stage, the second non-REM stage, the third non-REM stage, and/or the REM) and the wakefulness stage. The sleep blocks can be calculated at a resolution of, for example, 30 seconds.

In some implementations, the systems and methods described herein can include generating or analyzing a hypnogram including a sleep-wake signal to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof based at least in part on the sleep-wake signal of a hypnogram.

In other implementations, one or more of the sensors 130 can be used to determine or identify the enter bed time (t_{bed}), the go-to-sleep time (t_{GTS}), the initial sleep time (tₛₗₑₑₚ), one or more first micro-awakenings (e.g., MA₁ and MA₂), the wake-up time (t_{wake}), the rising time (tᵣᵢₛₑ), or any combination thereof, which in turn define the sleep session. For example, the enter bed time t_{bed} can be determined based on, for example, data generated by the motion sensor 138, the microphone 140, the camera 150, or any combination thereof. The go-to-sleep time can be determined based on, for example, data from the motion sensor 138 (e.g., data indicative of no movement by the user), data from the camera 150 (e.g., data indicative of no movement by the user and/or that the user has turned off the lights), data from the microphone 140 (e.g., data indicative of the using turning off a TV), data from the user device 170 (e.g., data indicative of the user no longer using the user device 170), data from the pressure sensor 132 and/or the flow rate sensor 134 (e.g., data indicative of the user turning on the respiratory therapy device 122, data indicative of the user donning the user interface 124, etc.), or any combination thereof.

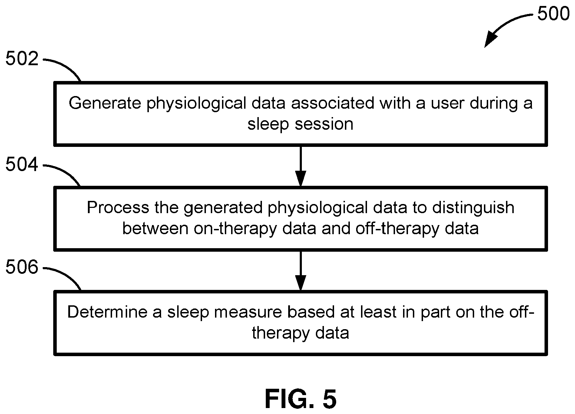

Referring to FIG. 5, a method 500 for determining a sleep measure for a user according to some implementations of the present disclosure is illustrated. One or more steps or aspects of the method 500 can be implemented using any portion or aspect of the system 100 described herein.

Step 502 of the method 500 includes generating physiological data associated with a user using a sleep session. The physiological data can be generated by, and received from, one or more of the sensors 130 (FIG. 1) described herein. The received physiological data can be indicative of one or more physiological parameters such as, for example, movement, heart rate, heart rate variability, cardiac waveform, respiration rate, respiration rate variability, respiration depth, a tidal volume, an inspiration amplitude, an expiration amplitude, an inspiration volume, an expiration volume, an inspiration-expiration ratio, perspiration, temperature (e.g., body temperature, core body temperature, surface temperature, etc.), blood oxygenation, photoplethysmography, pulse transit time, blood pressure, or any combination thereof. The physiological data can be received from at least one of the one or more sensors 130 by, for example, the electronic interface 119 and/or the user device 170 described herein, and stored in the memory 114 (FIG. 1). The physiological data can be received by the electronic interface 119 or the user device 170 from at least one of the one or more sensors 130 either directly or indirectly (e.g., with one or more intermediaries).

In some implementations, at least one of the one or more sensors 130 generates physiological data, such as activity-related physiological data, associated with the user outside the sleep session. For example, physiological data can be generated or obtained during a sleep session by a first sensor that is coupled to or integrated in the respiratory therapy system 120, the user device 170 or the activity tracker 180 as described herein, while further physiological data, such as activity-related physiological data, can be generated or obtained outside of a sleep session by the first sensor or by a second sensor is that is coupled to or integrated in the user device 170 or the activity tracker 180. The user may have the user device 170 or the activity tracker 180 on during a normal daily routine, and the user device 170 or the activity tracker 180 can generate the physiological data while the user is awake. The activity-related physiological data can be used to determine an activity measurement associated with the user, such as, for example, number of steps, a distance traveled, a number of steps climbed, a duration of physical activity, a type of physical activity, an intensity of physical activity, time spent standing, a respiration rate, an average respiration rate, a resting respiration rate, a respiration rate variability, a heart rate, an average heart rate, a resting heart rate, a maximum heart rate, a heart rate variability, a number of calories burned, blood oxygen saturation, electrodermal activity (also known as skin conductance or galvanic skin response), or any combination thereof. In some implementations, the user device 170 or the activity tracker 180 electronic devices can be used to generate the physiological data during the sleep session. In some implementations, the respiratory therapy system 120 can, additionally or alternatively, be used to generate the physiological data.

Step 504 of the method 500 includes processing the generated physiological data of step 502 to distinguish between on-therapy data and off-therapy data. On-therapy data is physiological data generated while the user is coupled to the respiratory therapy system 120 (i.e., the user is wearing a user interface which is connected via a conduit to a respiratory therapy device of the respiratory therapy system), and the respiratory therapy system 120 is supplying pressurized air to an airway of the user. Off-therapy data, on the other hand, is physiological data generated while the user is asleep and the respiratory therapy system 120 is not supplying pressurized air to the airway of the user, and during which the user may or may not be coupled to the respiratory therapy system 120. On-therapy data will usually be generated when the user is using the respiratory therapy system 120, adhering to a prescribed therapy for treating respiratory and/or sleep-related disorders (e.g., OSA, SDB, etc.). Off-therapy data is generated outside of this setting. For example, physiological data generated while the user is asleep but not using the respiratory therapy system 120 is off-therapy data. In some implementations, off-therapy data can include physiological data generated while the user is awake.

In some implementations, during a sleep session, the system 100 can generate both on-therapy and off-therapy data. There can be one or more periods within the sleep session where the user is not using the respiratory therapy system 120 for treating her sleep-related or respiratory disorder. In an example, the user can interface with the respiratory therapy system 120 and go to sleep at her bedtime, and during the night, can wake up for a bathroom break. The user can forget to, or decide not to, use the respiratory therapy system 120 when she goes back to bed after the bathroom break. In this example, during a first part of the sleep session, the user used the respiratory therapy system 120, and during a second part of the sleep session, the user forgot to, or decided not to, use the respiratory therapy system 120. Therefore, the system 100 can generate on-therapy data during the first part of the sleep session and off-therapy data during the second part of the sleep session. Although described in the context of two parts of the sleep session, this can be extended to more than two parts to the sleep session, depending on how many times the user dons and removes the user interface 124.

Using the respiratory therapy system 120 is an effective treatment to combat effects related to respiratory and/or sleep-related disorders. For some users, the respiratory therapy system 120 produces dramatic positive results, however some users may not notice a more subtle positive effect in treatment. Sometimes users may not be diligent in using the respiratory therapy system 120 when positive effects are not noticeable. For example, the user that does not notice positive effects can wake in the middle of the night and not use the respiratory therapy system 120 when going back to sleep. In some cases, the user may forget to use the respiratory therapy system 120 altogether, skipping a night or multiple nights. Sometimes, the user may feel that the respiratory therapy system 120 is not necessary to get good sleep, and diligence can wane over time. The one or more sensors 130 can monitor the user not only when the user is using the respiratory therapy system 120 but also when the user is not using the respiratory therapy system 120. Preferably, during sleep sessions where the user is not using the respiratory therapy system 120 or partially uses the respiratory therapy system 120, it can be advantageous to have methods of monitoring quality of sleep of the user. Thus, the activity tracker 180 and/or the user device 170 associated with the user continues accumulating physiological data even when the respiratory therapy system 120 is turned off or is uncoupled from the user.

The respiratory therapy system 120 may further generate second physiological data related to one or more comorbidities experienced by the user 210. This second physiological data may be generated by one or more sensors integrated and/or coupled to the respiratory therapy system 120, such as pressure sensor 132, flow rate sensor 134, temperature sensor 136, etc. Additionally or alternatively, this second physiological data may be generated by at least one or more of sensors comprised in the activity tracker 180 and/or the user device 170, such as sensors 130, or any other suitable sensor. It is further contemplated that the second physiological data may be generated from subjective input from, for example, the user, physician or caregiver. In some implementations, the one or more sensors can include a blood pressure sensor, for measuring and/or monitoring a user's blood pressure. Specific examples include a nocturnal blood pressure sensor for measuring and/or monitoring a user's blood pressure during a sleep session. The blood pressure sensor can measure, for example, a systolic blood pressure component and/or a diastolic blood pressure component. The blood pressure sensor may be a sphygmomanometer including an inflatable cuff that can be worn by a user and a pressure sensor. The blood pressure sensor can monitor comorbidities such as hypertension. The blood pressure sensor can be communicatively coupled with, and/or physically integrated in (e.g., within a housing), the control system 110, the memory 114, the respiratory therapy system 120, the user device 170, activity tracker 180 and/or a smart device such as a smartphone. In implementations, the one or more comorbidities include cardiovascular diseases such as coronary artery disease, arrhythmias, etc. which may be detected and/or monitored by one or more tertiary sensors such as a photoplethysmogram (PPG) sensor, an electrocardiogram (ECG) sensor, etc. In implementations, the one or more sensors can include a blood glucose sensor for measuring and/or monitoring a user's blood levels. Specific examples include a continuous glucose sensor, which may be worn by a user in or on the skin and which typically measures the level of glucose in interstitial fluid at predetermined time intervals as an indication of blood glucose levels. The one or more comorbidities which may be detected and/or monitored by such one or more sensors include metabolic disorders such as diabetes mellitus. In further implementations, the one or more sensors include a blood oxygen saturation (SpO2) sensor, spirometer, an analyte sensor (such as for the measurement and/or monitoring of expired carbon dioxide, nitric oxide (NO), and/or carbon monoxide (CO)), or any combination thereof. The one or more comorbidities which may be detected and/or monitored by such one or more tertiary sensors include respiratory diseases such as COPD and asthma. The generation of the second physiological data related to one or more comorbidities allows the user, treating physician, and/or other stakeholders to correlate the incidence and/or progression of the one or more comorbidities with the physiological data associated with the user during one or more sleep sessions and/or to correlate the incidence and/or progression of the one or more comorbidities with the user's use of the respiratory therapy. In this way, the incidence, progression (e.g. improvement, deterioration, or no change in), or both the incidence and progression, of the one or more comorbidities can be associated with parameters such as quality of sleep, adherence to therapy, etc. as monitored when the user interface 124 is engaged with the user and when the user interface 124 is not engaged with the user. Furthermore, the sleep score or therapy score disclosed herein can include as an input the physiological data related to one or more comorbidities, and can further include as an output how the use or non-use of the respiratory therapy system 120 has impacted the incidence and/or progression of the one or more comorbidities.

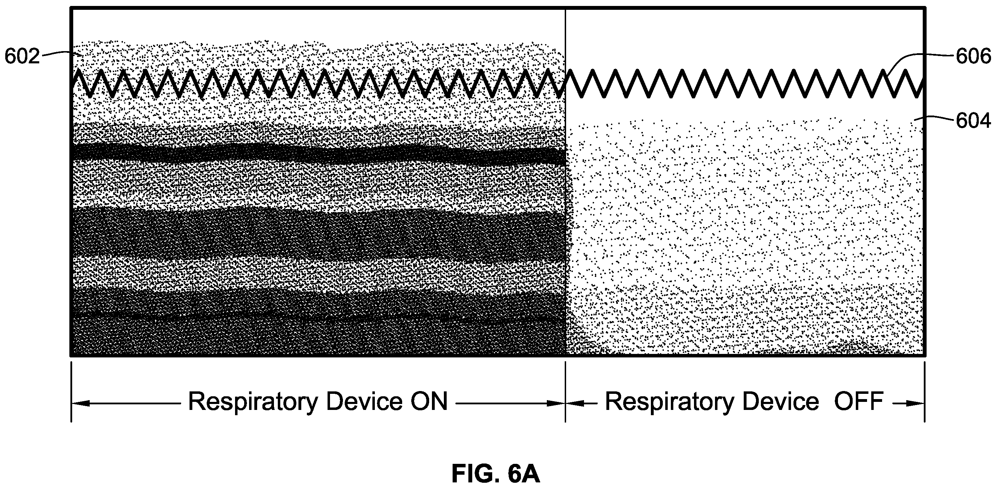

In some implementations, at step 504, on-therapy data and off-therapy data can be distinguished based on audio data generated by the microphone 140. When the respiratory therapy system 120 is running, noise from the respiratory therapy system 120 changes characteristic of environmental noise in the user's bedroom or sleeping area. The noise from the respiratory therapy system 120 can include motor tones, white noise due to pressurized air being generated by the respiratory therapy system 120, etc. An example of these audio artifacts is illustrated in FIG. 6A, according to some implementations of the present disclosure. In FIG. 6A, when the respiratory therapy system 120 is turned on, the ambient noise is at a higher level as indicated by the level 602, but when turned off, the ambient noise is at a lower level as indicated by level 604. In FIG. 6A, the x-axis represents time and the y-axis represents frequency. In some implementations, the physiological data obtained while the audio data from the microphone 140 indicates a noise floor lower than the higher noise floor (e.g., the level 604) is classified as off-therapy data.

In some implementations, on-therapy data may be classified as such if the physiological data is obtained while the audio data from the microphone 140 indicates a higher noise floor (e.g., the level 602). Respiratory therapy devices, after a user interface has been removed, can automatically switch off (or go on standby) or reduce airflow from the flow generator (often in the form of light puffs of air, which can ramp up to therapy pressure if the user interface is replaced on the face) prior to switching off, which actions produce distinctive noises which may be detected by the disclosed system. In implementations, the on-therapy data and off-therapy data can be distinguished, or further distinguished, based on audio data generated by the microphone 140 which is indicative of breathing of the user while wearing a user interface, i.e. breathing through a user interface is distinct from breathing without a user interface to the atmosphere. In other implementations, the on-therapy data and off-therapy data can be distinguished, or further distinguished, based on audio data generated by the microphone 140 which is indicative of the user exiting the bed and/or returning to bed. In an example, the user may remove the user interface and leave the bed resulting in greater than noise when leaving the bed and reduced noise when the respiratory therapy device switches off or there is reduced airflow from the flow generator. The user may subsequently return to the bed and don the user interface resulting in greater than ambient noise when returning to the bed and when the respiratory therapy device switches on or there is increased airflow from the flow generator.

In some implementations, the acoustic sensor 141 is used to obtain physiological data, and sounds associated with the acoustic sensor 141 are captured. For example, frequency modulated continuous wave (FMCW) waveform 606 can be distinguished from the background ambient noise. The FMCW waveform 606 can be swept up and down between 18 kHz and 20 kHz. In some implementations, there are usually 16 triangles per second, with each triangular chirp including around 3000 samples at an audio sampling rate of 48 kHz.

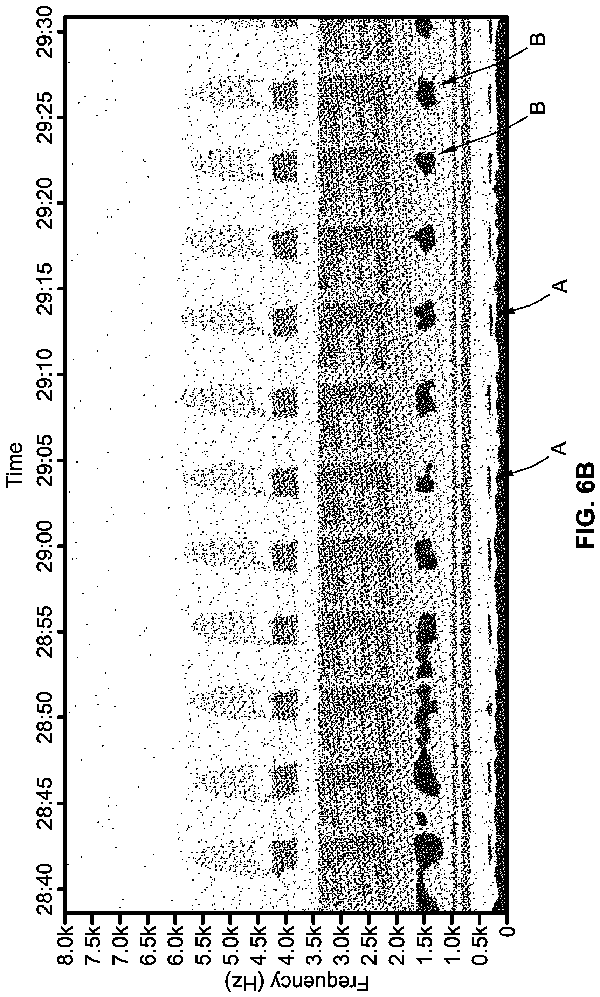

Artifacts can exist in a captured audio signal which indicate that (i) the respiratory therapy device 122 is running, (ii) the user 210 is wearing the user interface 124 (thus, receiving therapy), or both (i) and (ii). FIG. 6B illustrates a captured audio signal over time that includes audio artifacts, according to some implementations of the present disclosure. In FIG. 6B, a tone indicated with "A" can be observed in the captured audio signal. The tone A is associated with exhalation of the user 210. Another tone (indicated with "B") can be detected in the captured audio signal. The tone B is indicative of a leak of pressurized air from a mask (i.e., a leak in the user interface 124). The leak in the mask indicates that the respiratory therapy device 122 is running and the user 210 is wearing the user interface 124 and receiving therapy. Advantageously, tones and/or features (e.g., tone A and tone B) are identifiable even if there are other sounds (e.g., snoring) in the environment of the user 210. The tones and/or features can be separated by frequency as shown in FIG. 6B.

In other aspects, operation/running of a respiratory therapy device 122 includes operating modes which produce distinctive audio signals. For example, the respiratory therapy device 122 can include an expiratory pressure relief (EPR) feature, which maintains an optimal treatment for the patient (i.e., the user 210) during inhalation and reduces pressure during exhalation, making it easier for the user 210 to breathe out, and which produces distinctive audio signals detectable by an acoustic sensor such as acoustic sensor 141.

In some implementations, the generated physiological data at step 502 is used to determine whether the user is asleep since time the user gets in bed may differ from the time the user actually falls asleep, as discussed in connection with FIG. 3. The one or more sensors 130 can, for example, listen for a change in breathing of the user, where rhythmic deeper breaths can indicate that the user is asleep. In some implementations, snoring events can be used as a marker to indicate that the user is asleep. In some implementations, motion data can be indicative of the user being asleep especially if the user does not change or switch positions for long durations. In some implementations, changes in heart rate of the user, changes in heart rate variability of the user, changes in core temperature of the user, changes in EEG signals associated with the user, or any combination thereof, can be used to determine whether the user is asleep. In some implementations, at least one of the sensors 130 included in the respiratory therapy system 120 provides flow signals indicative of the breathing of the user such that the change in breathing pattern of the user can be used to determine that the user is asleep.

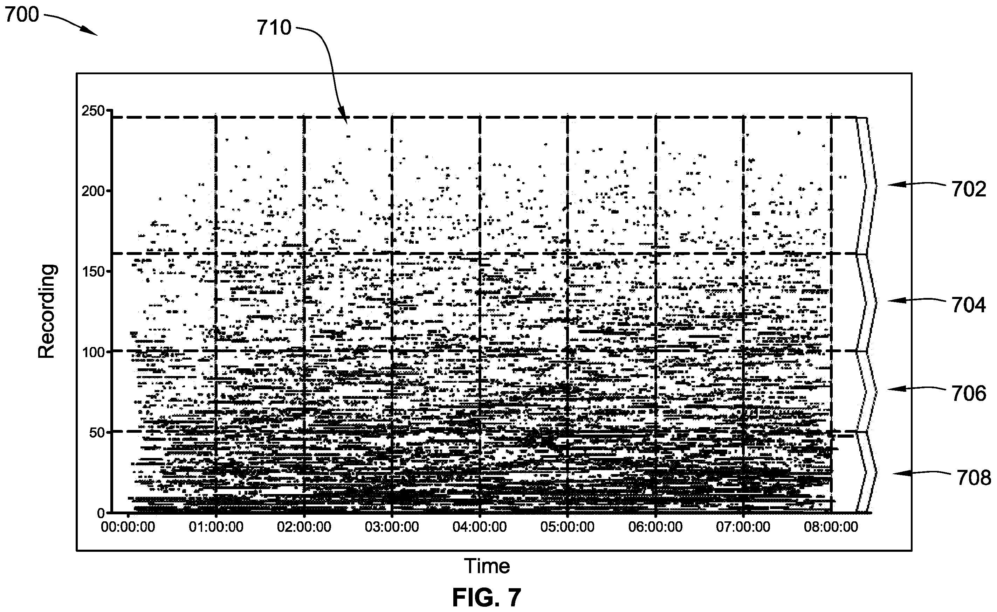

In some implementations, at step 504, a pattern of events, or a pattern of clustered events, can be used to distinguish between on-therapy data and off-therapy data. Events include apneas, hypopneas, or any other events or symptoms associated with poor sleep as discussed above in connection with FIG. 1. In an example, FIG. 7 illustrates a graph 700 showing a mapping of events according to some implementations of the present disclosure. The graph 700 illustrates multiple patterns of events for different individuals while each of the individuals is asleep. Each number on the y-axis of the graph 700 labeled "Recording" indicates a specific individual, and the x-axis of the graph 700 labeled "Time" indicates time during a sleep session. Each dot (e.g., dot 710) indicates an event that occurred at a specific time. Some dots are close in succession, and based on the resolution of the graph 700, can appear as a line. The different individuals can be grouped based on severity or frequency of events. The graph 700 includes group 702, group 704, group 706, and group 708. Individuals in the group 702 experience events that are not very close to each other, hence are sparse in time and low in number across the sleep session. Individuals in the group 708 experience events that are close to each other and more frequent compared to the group 702 and high in number across the sleep session. These individuals in the groups 704 and 706 experience events at a number and frequency less than the group 708 but greater than the group 706. In some implementations, individuals in the group 702 have AHI values between 0 and 5, individuals in the group 704 have AHI values between 5 and 15, individuals in the group 706 have AHI values between 15 and 30, and individuals in the group 708 have AHI values greater than 30. In the example where the user wakes in the middle of the night and forgets to use the respiratory therapy system 120, cluster of events can help separate on-therapy data from off-therapy data.

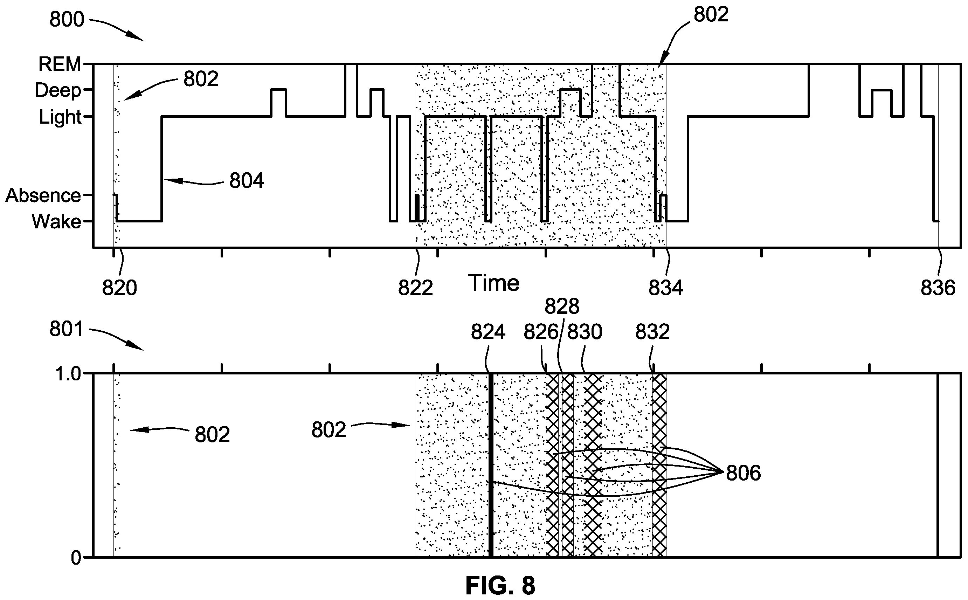

Referring to FIG. 8, a hypnogram 800 and a corresponding events graph 801 are provided for an individual, according to some implementations of the present disclosure. The hypnogram 800 displays a sleep-wake signal 804 over time during an example sleep session. The sleep-wake signal 804 traces through whether physiological data is being measured (Absence), whether the individual is awake (Wake), or whether the individual is in any one of a light sleep stage, a deep sleep stage, or a REM sleep stage. The events graph 801 indicates one or more clustered events 806 for the individual during the sleep session. During the sleep session, the individual is off-therapy, and portions of the sleep session where the individual is off-therapy is indicated by label 802 (e.g., between time 822 and time 834).

In the hypnogram 800, at time 820, the individual is in bed but awake (as indicated by the sleep-wake signal 804). The individual falls asleep, enters light sleep (as indicated by the sleep-wake signal 804), and is on-therapy while asleep. Prior to time 822, the individual wakes from sleep, and from time 822 to time 834, the individual falls back asleep but is off-therapy. After time 834, the individual wakes up to use the respiratory therapy system 120, thus going back on-therapy. At time 836, the individual wakes for the day, thus ending the sleep session.

In the events graph 801, clustered events 806 are shown to occur during the off-therapy portion of the sleep session indicated by the label 802. A first set (e.g., cluster) of events begins at time 824, a second set of events begins at time 826, a third set of events begins at time 828, a fourth set of events begins at time 830, and a fifth set of events begins at time 832. The first set of events last for a shorter duration when compared to any of the other sets of events, although this is not always the case. The events graph 801 for the individual allows the control system 110 to determine the presence of the clustered events 806. The hypnogram 800 and the events graph 801 allows the control system 110 to determine the presence of the clustered events 806 and during which sleep state or sleep stage the events occur. Advantageously, this provides greater confidence that the individual is off-therapy. In some implementations, the presence of the clustered events 806 is used to distinguish between off-therapy data and on-therapy data. In some implementations, since more events are expected to occur off-therapy when compared to on-therapy, the control system 110 can use a threshold to determine that clustered events 806 that exceed a certain frequency and/or duration indicate that the individual is off-therapy. In some implementations, the control system 110 can use the spacing between clustered events 806 to determine that the individual is off-therapy.

In some implementations, the respiratory therapy device 122 being on or off can be determined based on detecting of airflow and/or sound intensity of pressurized air from the respiratory therapy device 122. The detected airflow and/or sound intensity can be used to determine whether the user interface 124 is coupled to the user. If the user interface 124 is coupled to the user, then the data collected is determined to be on-therapy data. In some implementations, types of events can be used to distinguish between on-therapy and off-therapy data. For example, the control system 110 can deduce from physiological data that the user is experiencing an apnea or a hypopnea event. If the user usually does not experience such events during therapy, then the control system 110 can determine that the user is off-therapy if such events, or a greater number of such events, are detected. In some implementations, the activity tracker 180 and/or the user device 170 can pick up audio data indicating leakage from the user interface 124 and thus determine that the user is on-therapy.

In some implementations, the pattern of events or the pattern of clustered events can be combined with other methods discussed herein to increase confidence or increase accuracy while distinguishing between on-therapy data and off-therapy data. For example, audio data from the microphone 140 can indicate that the respiratory therapy device 122 is on and the control system 110 can determine that on-therapy data is being collected. This determination can be later cross-checked with the pattern of events. If the pattern of events, as discussed with respect to FIG. 7, indicates a severity of events that matches a severity when the user is on-therapy, then a confidence in classifying the user as being on-therapy is increased. If the pattern of events indicates otherwise, then the confidence is decreased.

Step 506 of the method 500 determining a sleep measure for the user based at least in part on the off-therapy data of step 504. The sleep measure is an indication of quality of sleep of the user during the sleep session, which quality of sleep may be based on, for example, duration of sleep, sleep architecture, etc. Additionally, or alternatively, the sleep measure is an indication of efficacy of therapy experienced by the user during the sleep session, which efficacy of therapy may be based on, for example, AHI, pattern of events, etc. In some implementations, the sleep measure is based at least in part on on-therapy data. Off-therapy data for the sleep session is generated for a duration where the user is asleep and not using the respiratory therapy system 120 for therapeutic purposes during the sleep session. On-therapy data for the sleep session is generated for a duration where the user is asleep and is using the respiratory therapy system 120 for therapeutic purposes during the sleep session. Therefore, during the sleep session, a total sleep time for the user can include an on-therapy sleep duration and/or an off-therapy sleep duration. The on-therapy sleep duration is a sleep duration where the user is using the respiratory therapy system 120. The off-therapy sleep duration is a sleep duration where the user is not using the respiratory therapy system 120. On-therapy sleep duration and off-therapy sleep duration can be determined from on-therapy data and off-therapy data, respectively.

On-therapy sleep duration and off-therapy sleep duration can be used to segment the total sleep time. An on-therapy sleep measure can be determined for the on-therapy sleep duration using the on-therapy data, and an off-therapy sleep measure can be determined for the off-therapy sleep duration using the off-therapy sleep data. The on-therapy sleep measure is an indication of sleep quality for the user during the on-therapy sleep duration, and the off-therapy sleep measure is an indication of sleep quality for the user during the off-therapy sleep duration. In some implementations where the total sleep time includes both off-therapy sleep duration and on-therapy sleep duration, the sleep measure determined at step 506 is a weighted combination of the on-therapy sleep measure and the off-therapy sleep measure.

In some implementations, the weighted combination is calculated as: $SM = \left({SM}_{ON}\times {T}_{ON}+{SM}_{OFF}\times {T}_{OFF}\right) / {T}_{SLEEP} ,$ where SM is sleep measure, *SM_{ON}* is on-therapy sleep measure, *SM_{OFF}* is off-therapy sleep measure, *T_{ON}* is on-therapy sleep duration, *T_{OFF}* is off-therapy sleep duration, and *T_{SLEEP}* is total sleep time. *T_{SLEEP}* = *T_{ON}* + *T_{OFF}.* Determining the sleep measure using the weighted combination expression is possible even in cases where there is only one of off-therapy data or on-therapy data. That is, during the sleep session, the user may not use the respiratory therapy system 120 at all while sleeping and thus there are no on-therapy data, or the user may use the respiratory therapy system 120 for the total sleep time and thus there are no off-therapy data. In such cases, either *T_{ON}* is set to zero or *T_{OFF}* is set to zero, and the sleep measure takes on the value of *SM_{ON}* or *SM_{OFF},* respectively.

In some implementations, the sleep measure includes an AHI value for the user. The off-therapy sleep measure can be an off-therapy AHI value, and the on-therapy sleep measure can be an on-therapy AHI value. For the user suffering from respiratory or sleep-related disorders, the off-therapy AHI is expected to be higher than the on-therapy AHI value.

In some implementations, the sleep measure includes one or more sleep-related parameters based at least in part on the physiological data of step 502. The one or more sleep-related parameters can include, for example, an identification of one or more events experienced by the user, a number of events per hour, a pattern of events, a total sleep time, a total time in bed, a wake-up time, a rising time, a hypnogram, a total light sleep time, a total deep sleep time, a total REM sleep time, a number of awakenings, a sleep-onset latency, or any combination thereof. The event(s) can include snoring, apneas, central apneas, positional apneas, obstructive apneas, mixed apneas, hypopneas, a mask leak (e.g., from the user interface 124), a restless leg, a sleeping disorder, choking, an increased heart rate, labored breathing, an asthma attack, an epileptic episode, a seizure, or any combination thereof. In some implementations, the sleep-related parameters can include a sleep score, such as the ones described in International Publication No. WO 2015/0063 64.

In some implementations, the determined sleep measure is calibrated. For example, the respiratory therapy system 120 includes at least a portion of the sensors 130, such as pressure sensor 132, flow rate sensor 134, and microphone 140, and can be used to determine the on-therapy sleep measure for the sleep session. The respiratory therapy system 120 can generate physiological data including a breathing pattern of the user which includes a respiration rate, a respiration depth, respiration variability, or any combination thereof. Alongside the respiratory therapy system 120, the user device 170 and/or the activity tracker 180 can be used to determine the on-therapy sleep measure as well. The determined on-therapy sleep measure from the user device 170 and/or the activity tracker 180 can be adjusted based at least in part on the on-therapy sleep measure determined by the respiratory therapy system 120. In some implementations, a linear factor relates both on-therapy sleep measures such that the on-therapy sleep measure determined by the respiratory therapy system 120 is divided by the on-therapy sleep measure determined by the user device 170 and/or the activity tracker 180 to determine the linear factor. In some implementations, the linear factor is calculated and regressed over multiple sleep sessions (e.g., two sleep sessions, five sleep sessions, etc.) to find an average factor that captures any fluctuations between the on-therapy sleep measure determined by the respiratory therapy system 120 and the on-therapy sleep measure determined by the user device 170 and/or the activity tracker 180.

In some implementations, the system 100 is geared to encouraging the user to be more diligent in using the respiratory therapy system 120 while sleeping. A communication can be sent for informing the user of the determined sleep measure (step 506), which may comprise the on-therapy sleep measure, the off-therapy sleep measure, or both. For example, the communication can be sent to the user device 170 to inform the user of the determined sleep measure (step 506). The user device 170 can display the determined sleep measure on the display device 172, the respiratory therapy system 120 can display the sleep measure on the display device 128. The communication can be audio broadcast through the speaker 142 integrated on any one of the user device 170, the activity tracker 180, and/or the respiratory therapy system 120. In some implementations, the communication can be a visual light indicator with multiple levels that indicate different colors for different interpretations of the determined sleep measure. For example, different colors can indicate a good sleep measure, a bad sleep measure, or a neutral sleep measure. An as illustrative example, the on-therapy sleep measure may be indicated in a particular color to indicate a good (positive) sleep measure, and the off-therapy sleep measure may be indicated in a different color to indicate a bad (negative) sleep measure.

In some implementations, the communication can include a recommendation for the user for a future sleep session. For example, the communication can include a recommendation that the user increase the on-therapy sleep duration in the future sleep session when compared to the on-therapy sleep duration for the most recent sleep session that the sleep measure pertains. The communication can include a recommendation that the user increase the on-therapy sleep duration in the future sleep session when compared to the off-therapy sleep duration for the most recent sleep session that the sleep measure pertains. In some implementations, the communication can include a recommendation that the user increase total sleep duration in the future sleep session.

In some implementations, an estimated sleep measure for the future sleep session can be determined based at least in part on an estimated total sleep duration for the user for the future sleep session. For example, the estimated total sleep duration can be based on an average sleep duration for the user during a time period. The time period can be a day, a couple of days, a week, a month, one or more weekdays, one or more days in a weekend, etc. In an example, if the future sleep session is a Monday, then a total sleep duration for a previous sleep session on a Monday can be used as the estimated total sleep duration.

The estimated sleep measure for the future sleep session can further be based at least in part on the on-therapy sleep measure for the most recent sleep session and/or the off-therapy sleep measure for the most recent sleep session. The estimated sleep measure can be a weighted combination of the estimated on-therapy sleep measure, the estimated off-therapy sleep measure, and portions of the estimated total sleep duration that the user is on therapy and portions of the estimated sleep duration that the user is off therapy.

In some implementations, the estimated sleep measure for the future sleep session is included in the communication provided to the user. The estimated sleep measure can be provided to the user to help encourage the user to be more diligent in using the respiratory therapy system 120. In some implementations, the portions of the estimated total sleep duration that the user is on therapy and portions of the estimated sleep duration that the user is off therapy are adjusted based on the most recent sleep session information. For example, if the total sleep time is 8 hours and the user spent 5 hours on therapy and 3 hours off therapy, then the portion of the estimated sleep duration that the user is on therapy can be adjusted upwards to 6 hours, 7 hours or 8 hours and the portion of the estimated sleep duration that the user is off therapy can be adjusted downwards in proportion to 2 hours, 1 hour, or 0 hours. These values can be used in the weighted combination to provide the estimated sleep duration for the future sleep session to show much the determined sleep session can improve if the user increased her on-therapy sleep duration and decreased her off-therapy sleep duration. Conversely, a similar calculation can be made to show the user detrimental effects on the sleep measure if on-therapy sleep duration were to be reduced and off-therapy sleep duration were to be increased.

In some implementations, a target sleep measure for the future sleep session is used to determine how much the user should increase her on-therapy sleep duration for the future sleep session and how much the user should decrease her off-therapy sleep duration for the future sleep session. In some implementations, the following expression is solved for to determine the amount to increase the on-therapy sleep duration and the amount to decrease the off-therapy sleep duration: ${SM}_{TARGET} = \left({SM}_{ON}\times \left({T}_{ON}+{T}_{X}\right)+{SM}_{OFF}\times \left({T}_{OFF}-{T}_{X}\right)\right) / {T}_{SLEEP} ,$ where *SM_{TARGET}* is the target sleep measure, *SM_{ON}* is on-therapy sleep measure, *SM_{OFF}* is off-therapy sleep measure, *T_{ON}* is on-therapy sleep duration, *T_{OFF}* is off-therapy sleep duration, *T_{X}* is the amount to increase on-therapy sleep duration (and decrease off-therapy sleep duration) in the future sleep session, and *T_{SLEEP}* is total sleep time. *T_{SLEEP}* = *T_{ON}* + *T_{OFF}.* For any given *SM_{TARGET},* the amount *T_{X}* can be solved for and communicated to the user. For example, the communication can include a recommendation instructing the user to use the respiratory therapy system 120 for an extra *T_{X}* duration in order to reach her target sleep measure. In some implementations, *T_{X} = T_{OFF},* thus eliminating any off-therapy sleep duration.

In some implementations, the total sleep time is not kept the same. That is, the amount to increase the on-therapy sleep duration is not equal to the amount to decrease the off-therapy sleep duration. In such implementations, an estimated total sleep duration is determined by adding the on-therapy sleep duration, the amount to increase the on-therapy sleep duration, and the off-therapy sleep duration, and subtracting the amount to decrease the off-therapy sleep duration.

Any one of the different sleep measures discussed herein can be included in the communication. For example, the communication can include the on-therapy sleep measure, the on-therapy sleep duration, the estimated sleep measure for the future sleep session, the target sleep measure for the future sleep session, the target sleep duration for the future sleep session, the target reduction of the off-therapy sleep duration, the target increase in on-therapy sleep duration, the estimated total sleep duration in the future sleep session, or any combination thereof.

One or more elements or aspects or steps, or any portion(s) thereof, from one or more of any of claims 1-98 below can be combined with one or more elements or aspects or steps, or any portion(s) thereof, from one or more of any of the other claims 1-98 or combinations thereof, to form one or more additional implementations and/or claims of the present disclosure.

While the present disclosure has been described with reference to one or more particular embodiments or implementations, those skilled in the art will recognize that many changes may be made thereto without departing from the spirit and scope of the present disclosure. Each of these implementations and obvious variations thereof is contemplated as falling within the spirit and scope of the present disclosure. It is also contemplated that additional implementations according to aspects of the present disclosure may combine any number of features from any of the implementations described herein.

## Claims

1. A method comprising:
generating (502), by a sensor (130), physiological data associated with a user (210) of a respiratory therapy system (120) during a sleep session;
generating, by a microphone (140), audio data associated with the user (210) of the respiratory therapy system (120) during the sleep session;
processing (504), by an electronic device including one or more processors (112), the generated physiological data to distinguish between on-therapy data and off-therapy data, wherein
(i) the on-therapy data is the generated physiological data while a respiratory therapy system (120) is coupled to the user (210) and supplies pressurized air to an airway of the user (210) via a user interface (124) worn by the user (210), and
(ii) the off-therapy data is the generated physiological data while the respiratory therapy system (120) is not supplying pressurized air to the airway of the user (210);
determining (506), by the electronic device, a sleep measure based at least in part on the off-therapy data;
**characterized in that** the method further comprises:
analyzing the audio data to distinguish between (i) the respiratory therapy system (120) operating and (ii) the respiratory therapy system (120) operating and supplying pressurized air to the airway of the user (210) via a user interface (124) worn by the user (210).

2. The method of claim 1, wherein the sensor (130) is included in the electronic device; and/or
the method further comprising generating, by the sensor (130), third physiological data associated with the user (210) outside of the sleep session.

3. The method of claim 1 or claim 2, wherein the physiological data includes sleep-related data, a number of events per hour, a pattern of events, a total sleep time, a total time in bed, a wake-up time, a rising time, a total light sleep time, a total deep sleep time, a total REM sleep time, a number of awakenings, a sleep-onset latency, respiration rate, heart rate, heart rate variability, temperature, or any combination thereof.

4. The method of any one of claims 1 to 3, further comprising:
determining, by the electronic device, that the user (210) is asleep based at least in part on the generated physiological data; and
optionally wherein the determining that the user (210) is asleep includes detecting a change in breathing of the user (210) based at least in part on the off-therapy data, detecting changes in movement of the user (210) based at least in part on the off-therapy data, detecting changes in heart rate of the user (210), detecting changes in heart rate variability of the user (210), detecting changes in core temperature of the user (210), detecting changes in electroencephalogram (EEG) signals associated with the user (210), or any combination thereof; and/or
wherein the determining that the user (210) is asleep includes determining that the respiratory therapy system (120) is supplying the pressured air to the airway of the user (210); and/or
wherein the determining that the user (210) is asleep includes determining that flow signals from the respiratory therapy system (120) are indicative of breathing of the user (210).

5. The method of any one of claims 1 to 4, wherein the sleep measure is apnea hypopnea index (AHI), sleep efficiency, number of sleep cycles, fragmentation index, sleep architecture, number and/or duration of sleep stages, number and/or duration of awakenings of the user (210), a total sleep time, a sleep onset latency, a wake-after-sleep-onset parameter, or any combination thereof; and/or
wherein the determining the sleep measure is further based at least in part on the on-therapy data

6. The method of any one of claims 1 to 5, further comprising:
determining an on-therapy sleep measure and an off-therapy sleep measure, the on-therapy sleep measure being determined from the on-therapy data and the off-therapy sleep measure being determined from the off-therapy data, wherein the determining the sleep measure is further based at least in part on the off-therapy sleep measure and the on-therapy sleep measure, and optionally wherein the on-therapy sleep measure is an on-therapy apnea hypopnea index (AHI) and the off-therapy sleep measure is an off-therapy AHI; and/or
determining an on-therapy sleep duration and an off-therapy sleep duration, the on-therapy sleep duration associated with the on-therapy sleep measure and the off-therapy sleep duration associated with the off-therapy sleep measure, and optionally wherein the determining the sleep measure is further based at least in part on the on-therapy sleep duration and the off-therapy sleep duration.

7. The method of any one of claims 1 to 6 further comprising:
determining an estimated sleep measure for a future sleep session based at least in part on an estimated total sleep duration of the user (210) for the future sleep session, and
optionally wherein the determining the estimated sleep measure is further based at least in part on an on-therapy sleep measure and an off-therapy sleep measure.

8. The method of any one of claims 1 to 7, further comprising:
determining an estimated on-therapy sleep measure for a portion of the sleep session that the respiratory therapy system (120) is not supplying pressurized air to the airway of the user (210); and/or
determining an estimated off-therapy sleep measure for a portion of the sleep session that the respiratory therapy system (120) is supplying pressurized air to the airway of the user (210); and/or
determining an estimated total sleep duration of the user (210) for a future sleep session based at least in part on a target sleep measure for the user (210) for the future sleep session, and optionally wherein the estimated total sleep duration includes an on-therapy sleep duration, an off-therapy sleep duration, or both, the on-therapy sleep duration being a duration that the user is asleep and coupled to the respiratory therapy system (120) and the off-therapy sleep duration being a duration that the user (210) is not receiving the pressurized air from the respiratory therapy system (120), and/or wherein the target sleep measure for the user (210) includes an on-therapy sleep measure, an off-therapy sleep measure, or both.

9. The method of any one of claims 1 to 8, wherein the on-therapy data includes physiological data received from the respiratory therapy system (120), and the method further comprises:
determining a respiratory sleep measure based on the physiological data received from the respiratory therapy system (120); and
adjusting the determined sleep measure based at least in part on the respiratory sleep measure, and optionally wherein the physiological data received from the respiratory therapy system (120) includes a breathing pattern of the user, the breathing pattern including a respiration rate, a respiration depth, respiration variability, or any combination thereof.

10. The method of any one of claims 1 to 9, further comprising:
causing, by the electronic device, a communication to be sent based at least in part on the determined sleep measure, and
optionally wherein the communication includes the sleep measure; and/or
wherein the communication includes a recommendation for the user (210) to (a) increase an on-therapy sleep duration compared to an off-therapy sleep duration, (b) increase the on-therapy sleep duration compared to a previous on-therapy sleep duration, or (c) both, the on-therapy sleep duration being a duration that the user is asleep and coupled to the respiratory therapy system (120) and the off-therapy sleep duration being a duration that the user (210) is asleep and not receiving the pressurized air from the respiratory therapy system (120); or
wherein the communication includes a recommendation for the user (210) to (a) decrease an off-therapy sleep duration compared to an on-therapy sleep duration, (b) decrease the off-therapy sleep duration compared to a previous off-therapy sleep duration, or (c) both, the on-therapy sleep duration being a duration that the user (210) is asleep and coupled to the respiratory therapy system (210) and the off-therapy sleep duration being a duration that the user (210) is asleep and not receiving the pressurized air from the respiratory therapy system (120), or
wherein the communication includes an on-therapy sleep measure, an on-therapy sleep duration, an off-therapy sleep measure, an off-therapy sleep duration, an estimated sleep measure for a future sleep session, a target sleep measure for the future sleep session, a target sleep duration for the future sleep session, a target reduction in off-therapy sleep duration, or a target increase in on-therapy sleep duration, or any combination thereof.

11. The method of claim 10, wherein the communication includes a recommendation for the user (210) to increase a total sleep duration.

12. The method of any one of claims 1 to 11, wherein a pattern of events, a clustering of events, or both are used to distinguish the off-therapy data and the on-therapy data; and/or
wherein noise associated with a motor of the respiratory therapy system (120), noise associated with movement of the user (210), or both are used to distinguish the off-therapy data and the on-therapy data; and/or
wherein noise associated with the user's (210) breathing while the respiratory therapy system (120) is coupled to the user (210) and supplying pressurized air to the airway of the user (210), noise associated with the user's (210) breathing while the respiratory therapy system (120) is not supplying pressurized air to the airway of the user (210), or both are used to distinguish the off-therapy data and the on-therapy data.

13. The method of any one of claims 1 to 12, further comprising:
analyzing audio data, generated by the sensor (130), to identify features indicative of exhalation by the user (210), features indicative of leaking of a user interface (124) of the respiratory therapy system (120), or both;
wherein the features indicative of the exhalation by the user (210) and the leaking of the user interface (124) of the respiratory therapy system (120), or both are used to distinguish the off-therapy data and the on-therapy data, and
optionally wherein physiological data generated when both the features indicative of the exhalation by the user (210) and the features indicative of the leaking of the user interface (124) of the respiratory therapy system (120) is included in the on-therapy data.

14. The method of any one of claims 1 to 13, further comprising generating second physiological data related to one or more comorbidities experienced by the user (210), and
optionally wherein the second physiological data is generated during the sleep session; and/or
wherein the incidence of the one or more comorbidities, the progression of the one or more comorbidities, or both are correlated with one or more of the sleep measure, the on-therapy sleep measure, the on-therapy sleep duration, the off-therapy sleep measure, and the off-therapy sleep duration

15. A system comprising:
a control system (110) including one or more processors (112);
a sensor (130); and
a memory (114) having stored thereon machine readable instructions;
wherein the control system (110) is coupled to the memory (114), and the system is configured to implement the method of any one of claims 1 to 14 when the machine executable instructions in the memory (114) are executed by at least one of the one or more processors (112) of the control system (110).

## Patentansprüche

1. Verfahren, umfassend:
Generieren (502), durch einen Sensor (130), physiologischer Daten, die mit einem Benutzer (210) eines Beatmungstherapiesystems (120) verknüpft sind, während einer Schlafsitzung;
Generieren, durch ein Mikrofon (140), von Audiodaten, die mit einem Benutzer (210) des Beatmungstherapiesystems (120) verknüpft sind, während der Schlafsitzung;
Verarbeiten (504), durch eine elektronische Vorrichtung, die eine oder mehrere Prozessoren (112) beinhaltet, der generierten physiologischen Daten zur Unterscheidung zwischen Daten während der Therapie und Daten außerhalb der Therapie, wobei
(i) die Daten während der Therapie die generierten physiologischen Daten sind, während ein Beatmungstherapiesystem (120) mit dem Benutzer (210) gekoppelt ist und über eine vom Benutzer (210) getragene Benutzerschnittstelle (124) Druckluft in die Atemwege des Benutzers (210) zuführt,
(ii) die Daten außerhalb der Therapie die generierten physiologischen Daten sind, während das Beatmungstherapiesystem (120) keine Druckluft in die Atemwege des Benutzers (210) zuführt;
Bestimmen (506), durch die elektronische Vorrichtung, eines Schlafmaßes, das zumindest teilweise auf den Daten außerhalb der Therapie basiert;
**dadurch gekennzeichnet, dass** das Verfahren ferner Folgendes umfasst:
Analysieren der Audiodaten, um zwischen (i) dem Beatmungstherapiesystem (120), das arbeitet, und (ii) dem Beatmungstherapiesystem (120), das arbeitet und dem Benutzer (210) über eine vom Benutzer (210) getragene Benutzerschnittstelle (124) Druckluft in die Atemwege zuführt, zu unterscheiden.

2. Verfahren nach Anspruch 1, wobei der Sensor (130) in die elektronische Vorrichtung beinhaltet ist; und/oder
das Verfahren ferner Generieren, durch den Sensor (130), von physiologischen Daten des Benutzers (210) außerhalb der Schlafsitzung umfasst.

3. Verfahren nach Anspruch 1 oder Anspruch 2, wobei die physiologischen Daten schlafbezogene Daten, eine Anzahl von Ereignissen pro Stunde, ein Ereignismuster, eine Gesamtschlafzeit, eine Gesamtzeit im Bett, eine Aufwachzeit, eine Aufstehzeit, eine Gesamtzeit von leichtem Schlaf, eine Gesamtzeit von Tiefschlaf, eine Gesamtzeit von REM-Schlaf, eine Anzahl von Aufwachvorgängen, eine Einschlaflatenz, eine Atemfrequenz, eine Herzfrequenz, eine Herzfrequenzvariabilität, eine Temperatur oder eine beliebige Kombination davon beinhalten.

4. Verfahren nach einem der Ansprüche 1 bis 3, ferner umfassend:
Bestimmen, durch die elektronische Vorrichtung, dass der Benutzer (210) schläft, zumindest teilweise basierend auf den generierten physiologischen Daten; und
wobei optional das Bestimmen, dass der Benutzer (210) schläft, Erkennen einer Änderung in Atmung des Benutzers (210) zumindest teilweise basierend auf den Daten außerhalb der Therapie, Erkennen von Änderungen in Bewegung des Benutzers (210) zumindest teilweise basierend auf den Daten außerhalb der Therapie, Erkennen von Änderungen in Herzfrequenz des Benutzers (210), Erkennen von Änderungen in Herzfrequenzvariabilität des Benutzers (210), Erkennen von Änderungen in Körperkerntemperatur des Benutzers (210), Erkennen von Änderungen in mit dem Benutzer (210) verknüpften Elektroenzephalogramm-(EEG)-Signalen oder eine beliebige Kombination davon beinhaltet; und/oder
wobei das Bestimmen, dass der Benutzer (210) schläft, Bestimmen beinhaltet, dass das Beatmungstherapiesystem (120) den Atemwegen des Benutzers (210) Druckluft zuführt; und/oder
wobei das Bestimmen, dass der Benutzer (210) schläft, Bestimmen beinhaltet, dass Flusssignale des Beatmungstherapiesystems (120) auf die Atmung des Benutzers (210) hinweisen.

5. Verfahren nach einem der Ansprüche 1 bis 4, wobei das Schlafmaß Apnoe-Hypopnoe-Index (AHI), Schlafeffizienz, Anzahl von Schlafzyklen, Fragmentierungsindex, Schlafarchitektur, Anzahl und/oder Dauer der Schlafphasen, Anzahl und/oder Dauer von Aufwachphasen des Benutzers (210), eine Gesamtschlafzeit, eine Einschlaflatenz, ein Parameter für Aufwachen-nach-Einschlafen oder eine beliebige Kombination davon ist; und/oder
wobei das Bestimmen des Schlafmaßes ferner zumindest teilweise auf den Daten während der Therapie basiert.

6. Verfahren nach einem der Ansprüche 1 bis 5, ferner umfassend:
Bestimmen eines Schlafmaßes während der Therapie und eines Schlafmaßes außerhalb der Therapie, wobei das Schlafmaß während der Therapie aus den Daten während der Therapie und das Schlafmaß außerhalb der Therapie aus den Daten außerhalb der Therapie bestimmt wird, wobei das Bestimmen des Schlafmaßes ferner zumindest teilweise auf dem Schlafmaß außerhalb der Therapie und dem Schlafmaß während der Therapie basiert, und wobei optional das Schlafmaß während der Therapie ein Apnoe-Hypopnoe-Index (AHI) während der Therapie ist und das Schlafmaß außerhalb der Therapie ein AHI außerhalb der Therapie ist; und/oder
Bestimmen einer Schlafdauer während der Therapie und einer Schlafdauer außerhalb der Therapie, wobei das Schlafmaß während der Therapie mit der Schlafdauer während der Therapie verknüpft ist und das Schlafmaß außerhalb der Therapie mit der Schlafdauer außerhalb der Therapie verknüpft ist, wobei optional das Bestimmen des Schlafmaßes ferner zumindest teilweise auf der Schlafdauer während der Therapie und der Schlafdauer außerhalb der Therapie basiert.

7. Verfahren nach einem der Ansprüche 1 bis 6, ferner umfassend:
Bestimmen eines geschätzten Schlafmaßes für eine zukünftige Schlafsitzung, basierend zumindest teilweise auf einer geschätzten Gesamtschlafdauer des Benutzers (210) für die zukünftige Schlafsitzung, und
wobei optional das Bestimmen des geschätzten Schlafmaßes zumindest teilweise auf einem Schlafmaß während der Therapie und einem Schlafmaß außerhalb der Therapie basiert.

8. Verfahren nach einem der Ansprüche 1 bis 7, ferner umfassend:
Bestimmen eines geschätzten Schlafmaßes während der Therapie für einen Teil der Schlafsitzung, in dem das Beatmungstherapiesystem (120) keine Druckluft in die Atemwege des Benutzers (210) zuführt; und/oder
Bestimmen eines geschätzten Schlafmaßes außerhalb der Therapie für einen Teil der Schlafsitzung, in dem das Beatmungstherapiesystem (120) den Atemwegen des Benutzers (210) Druckluft zuführt; und/oder
Bestimmen einer geschätzten Gesamtschlafdauer des Benutzers (210) für eine zukünftige Schlafsitzung basierend zumindest teilweise auf einem Ziel-Schlafmaß für den Benutzer (210) für die zukünftige Schlafsitzung, und wobei optional die geschätzte Gesamtschlafdauer eine Schlafdauer während der Therapie, eine Schlafdauer außerhalb der Therapie oder beides beinhaltet, die Schlafdauer während der Therapie eine Dauer ist, in der der Benutzer schläft und mit dem Beatmungstherapiesystem (120) gekoppelt ist, und die Schlafdauer außerhalb der Therapie eine Dauer ist, in der der Benutzer (210) keine Druckluft vom Beatmungstherapiesystem (120) empfängt, und/oder wobei das Ziel-Schlafmaß für den Benutzer (210) eine Schlafmaß während der Therapie, eine Schlafmaß außerhalb der Therapie oder beides beinhaltet.

9. Verfahren nach einem der Ansprüche 1 bis 8, wobei die Daten während der Therapie physiologische Daten beinhalten, die vom Beatmungstherapiesystem (120) empfangen werden, und das Verfahren ferner Folgendes umfasst:
Bestimmen eines respiratorischen Schlafmaßes basierend auf den vom Beatmungstherapiesystem empfangenen physiologischen Daten (120); und
Anpassen des bestimmten Schlafmaßes zumindest teilweise basierend auf dem respiratorischen Schlafmaß, und wobei optional die vom Beatmungstherapiesystem (120) empfangenen physiologischen Daten ein Atemmuster des Benutzers beinhalten, wobei das Atemmuster eine Atemfrequenz, eine Atemtiefe, eine Atemvariabilität oder eine beliebige Kombination davon beinhaltet.

10. Verfahren nach einem der Ansprüche 1 bis 9, ferner umfassend:
Veranlassen, durch die elektronische Vorrichtung, eine Mitteilung zu senden, die zumindest teilweise auf dem bestimmten Schlafmaß basiert, und
wobei optional die Mitteilung das Schlafmaß beinhaltet; und/oder
wobei die Mitteilung eine Empfehlung an den Benutzer (210) beinhaltet, (a) eine Schlafdauer während der Therapie im Vergleich zu einer Schlafdauer außerhalb der Therapie zu verlängern, (b) die Schlafdauer während der Therapie im Vergleich zu einer früheren Schlafdauer während der Therapie zu verlängern oder (c) beides, wobei die Schlafdauer während der Therapie eine Dauer ist, in der der Benutzer schläft und mit dem Beatmungstherapiesystem (120) gekoppelt ist, und die Schlafdauer außerhalb der Therapie die Dauer ist, in der der Benutzer (210) schläft und keine Druckluft vom Beatmungstherapiesystem (120) empfängt; oder
wobei die Mitteilung eine Empfehlung an den Benutzer (210) beinhaltet, (a) eine Schlafdauer außerhalb der Therapie im Vergleich zu einer Schlafdauer während der Therapie zu verkürzen, (b) die Schlafdauer außerhalb der Therapie im Vergleich zu einer vorherigen Schlafdauer außerhalb der Therapie zu verkürzen oder (c) beides, die Schlafdauer während der Therapie eine Dauer ist, in der der Benutzer (210) schläft und mit dem Beatmungstherapiesystem (210) gekoppelt ist, und die Schlafdauer außerhalb der Therapie die Dauer ist, in der der Benutzer (210) schläft und keine Druckluft vom Beatmungstherapiesystem (120) empfängt, oder
wobei die Mitteilung ein Schlafmaß während der Therapie, eine Schlafdauer während der Therapie, ein Schlafmaß außerhalb der Therapie, eine Schlafdauer außerhalb der Therapie, ein geschätztes Schlafmaß für eine zukünftige Schlafsitzung, ein Ziel-Schlafmaß für die zukünftige Schlafsitzung, eine Ziel-Schlafdauer für die zukünftige Schlafsitzung, eine Zielreduzierung der Schlafdauer außerhalb der Therapie oder eine Zielverlängerung der Schlafdauer während der Therapie oder eine beliebige Kombination davon beinhaltet.

11. Verfahren nach Anspruch 10, wobei die Mitteilung eine Empfehlung an den Benutzer (210) beinhaltet, eine Gesamtschlafdauer zu verlängern.

12. Verfahren nach einem der Ansprüche 1 bis 11, wobei ein Ereignismuster, eine Häufung von Ereignissen oder beides verwendet wird, um die Daten außerhalb der Therapie und die Daten während der Therapie zu unterscheiden; und/oder
wobei Geräusche, die mit einem Motor des Beatmungstherapiesystems (120) verknüpft sind, Geräusche, die mit einer Bewegung des Benutzers (210) verknüpft sind, oder beides verwendet werden, um die Daten außerhalb der Therapie und die Daten während der Therapie zu unterscheiden; und/oder
wobei Geräusche, die mit der Atmung des Benutzers (210) verknüpft sind, während das Beatmungstherapiesystem (120) mit dem Benutzer (210) gekoppelt ist und den Atemwegen des Benutzers (210) Druckluft zuführt, Geräusche, die mit der Atmung des Benutzers (210) verknüpft sind, während das Beatmungstherapiesystem (120) keine Druckluft in die Atemwege des Benutzers (210) zuführt, oder beides verwendet werden, um die Daten außerhalb der Therapie und die Daten während der Therapie zu unterscheiden.

13. Verfahren nach einem der Ansprüche 1 bis 12, ferner umfassend:
Analysieren von Audiodaten, die vom Sensor (130) generiert werden, um Merkmale, die auf Ausatmung des Benutzers (210) hinweisen, Merkmale, die auf ein Leck in einer Benutzerschnittstelle (124) des Beatmungstherapiesystems (120) hinweisen, oder beide zu identifizieren;
wobei die Merkmale, die auf die Ausatmung des Benutzers (210) und das Leck der Benutzerschnittstelle (124) des Beatmungstherapiesystems (120) oder beides hinweisen, dazu verwendet werden, die Daten außerhalb der Therapie und die Daten während der Therapie zu unterscheiden, und
wobei optional physiologische Daten, die generiert werden, wenn sowohl die Merkmale, die auf die Ausatmung des Benutzers (210) hinweisen, als auch die Merkmale, die auf ein Leck der Benutzerschnittstelle (124) des Beatmungstherapiesystems (120) hinweisen, in den Daten während der Therapie beinhaltet sind.

14. Verfahren nach einem der Ansprüche 1 bis 13, ferner umfassend Generieren zweiter physiologischer Daten in Bezug auf eine oder mehrere Komorbiditäten, die vom Benutzer (210) erlebt sind, und
wobei optional die zweiten physiologischen Daten während der Schlafsitzung generiert werden; und/oder
wobei das Auftreten der einen oder mehreren Komorbiditäten, das Fortschreiten der einen oder mehreren Komorbiditäten oder beides mit einem oder mehreren des Schlafmaßes, des Schlafmaßes während der Therapie, der Schlafdauer während der Therapie, des Schlafmaßes außerhalb der Therapie und der Schlafdauer außerhalb der Therapie korreliert sind.

15. System, umfassend:
ein Steuerungssystem (110), das einen oder mehrere Prozessoren (112) beinhaltet;
einen Sensor (130); und
einen Speicher (114), der darauf gespeicherte maschinenlesbare Anweisungen aufweist;
wobei das Steuerungssystem (110) mit dem Speicher (114) gekoppelt ist und das System so konfiguriert ist, dass es das Verfahren nach einem der Ansprüche 1 bis 14 implementiert, wenn die maschinenausführbaren Anweisungen im Speicher (114) von zumindest einem des einen oder der mehreren Prozessoren (112) des Steuerungssystems (110) ausgeführt werden.

## Revendications

1. Procédé comprenant :
la génération (502), par un capteur (130), de données physiologiques associées à un utilisateur (210) d'un système de thérapie respiratoire (120) pendant une séance de sommeil ;
la génération, par un microphone (140), de données audio associées à l'utilisateur (210) du système de thérapie respiratoire (120) pendant la séance de sommeil ;
le traitement (504), par un dispositif électronique incluant un ou plusieurs processeurs (112), des données physiologiques générées pour distinguer les données de thérapie des données hors thérapie, dans lequel
(i) les données de thérapie sont les données physiologiques générées lorsqu'un système de thérapie respiratoire (120) est connecté à l'utilisateur (210) et fournit de l'air sous pression aux voies respiratoires de l'utilisateur (210) via une interface utilisateur (124) portée par l'utilisateur (210), et
(ii) les données hors thérapie sont les données physiologiques générées lorsque le système de thérapie respiratoire (120) ne fournit pas d'air sous pression aux voies respiratoires de l'utilisateur (210) ;
la détermination (506), par le dispositif électronique, d'une mesure de sommeil sur la base au moins en partie des données hors thérapie ;
**caractérisé en ce que** le procédé comprend en outre :
l'analyse des données audio pour distinguer (i) le système de thérapie respiratoire (120) en fonctionnement et (ii) le système de thérapie respiratoire (120) en fonctionnement et fournissant de l'air sous pression aux voies respiratoires de l'utilisateur (210) via une interface utilisateur (124) portée par l'utilisateur (210).

2. Procédé selon la revendication 1, dans lequel le capteur (130) est inclus dans le dispositif électronique ; et/ou
le procédé comprenant en outre la génération, par le capteur (130), de troisièmes données physiologiques associées à l'utilisateur (210) en dehors de la séance de sommeil.

3. Procédé selon la revendication 1 ou la revendication 2, dans lequel les données physiologiques incluent des données relatives au sommeil, un nombre d'événements par heure, un profil d'événements, une durée totale de sommeil, une durée totale passée au lit, une heure de réveil, une heure de lever, une durée totale de sommeil léger, une durée totale de sommeil profond, une durée totale de sommeil paradoxal, un nombre de réveils, une latence d'endormissement, une fréquence respiratoire, une fréquence cardiaque, une variabilité de fréquence cardiaque, une température ou toute combinaison de ces éléments.

4. Procédé selon l'une quelconque des revendications 1 à 3, comprenant en outre :
la détermination, par le dispositif électronique, que l'utilisateur (210) dort, sur la base au moins en partie des données physiologiques générées ; et
éventuellement dans lequel la détermination que l'utilisateur (210) dort inclut la détection d'un changement de la respiration de l'utilisateur (210) sur la base au moins en partie des données hors thérapie, la détection de changements de mouvements de l'utilisateur (210) sur la base au moins en partie des données hors thérapie, la détection de changements de la fréquence cardiaque de l'utilisateur (210), la détection de changements de la variabilité de fréquence cardiaque de l'utilisateur (210), la détection de changements d'une température centrale de l'utilisateur (210), la détection de changements de signaux d'électroencéphalogramme (EEG) associés à l'utilisateur (210), ou toute combinaison de ces éléments ; et/ou
dans lequel la détermination que l'utilisateur (210) dort inclut la détermination que le système de thérapie respiratoire (120) fournit de l'air sous pression aux voies respiratoires de l'utilisateur (210) ; et/ou
dans lequel la détermination que l'utilisateur (210) dort inclut la détermination que des signaux de flux provenant du système de thérapie respiratoire (120) sont indicatifs de la respiration de l'utilisateur (210).

5. Procédé selon l'une quelconque des revendications 1 à 4, dans lequel la mesure de sommeil est un indice d'apnée-hypopnée (IAH), une efficacité de sommeil, un nombre de cycles de sommeil, un indice de fragmentation, une architecture de sommeil, un nombre et/ou une durée de phases de sommeil, un nombre et/ou une durée de réveils de l'utilisateur (210), une durée totale de sommeil, une latence d'endormissement, un paramètre de réveil après l'endormissement, ou toute combinaison de ces éléments ; et/ou
dans lequel la détermination de la mesure de sommeil est en outre sur la base au moins en partie des données de thérapie.

6. Procédé selon l'une quelconque des revendications 1 à 5, comprenant en outre :
la détermination d'une mesure de sommeil de thérapie et d'une mesure de sommeil hors thérapie, la mesure de sommeil de thérapie étant déterminée à partir des données de thérapie et la mesure de sommeil hors thérapie étant déterminée à partir des données hors thérapie, dans lequel la détermination de la mesure de sommeil est en outre sur la base au moins en partie de la mesure de sommeil hors thérapie et la mesure de sommeil de thérapie, et éventuellement dans lequel la mesure de sommeil de thérapie est un indice d'apnée-hypopnée (IAH) de thérapie et la mesure de sommeil hors thérapie est un IAH hors thérapie ; et/ou
la détermination d'une durée de sommeil de thérapie et d'une durée de sommeil hors thérapie, la durée de sommeil de thérapie étant associée à la mesure de sommeil de thérapie et la durée de sommeil hors thérapie étant associée à la mesure de sommeil hors thérapie, et éventuellement dans lequel la détermination de la mesure de sommeil est en outre sur la base au moins en partie de la durée de sommeil de thérapie et de la durée de sommeil hors thérapie.

7. Procédé selon l'une quelconque des revendications 1 à 6, comprenant en outre :
la détermination d'une mesure de sommeil estimée pour une future séance de sommeil sur la base au moins en partie d'une durée totale de sommeil estimée de l'utilisateur (210) pour la future séance de sommeil, et
éventuellement, dans lequel la détermination de la mesure de sommeil estimée est en outre sur la base au moins en partie d'une mesure de sommeil de thérapie et d'une mesure de sommeil hors thérapie.

8. Procédé selon l'une quelconque des revendications 1 à 7, comprenant en outre :
la détermination d'une mesure de sommeil de thérapie estimée pour une partie de la séance de sommeil pendant laquelle le système de thérapie respiratoire (120) ne fournit pas d'air sous pression aux voies respiratoires de l'utilisateur (210) ; et/ou
la détermination d'une mesure de sommeil hors thérapie estimée pour une partie de la séance de sommeil pendant laquelle le système de thérapie respiratoire (120) fournit de l'air sous pression aux voies respiratoires de l'utilisateur (210) ; et/ou
la détermination d'une durée totale de sommeil estimée de l'utilisateur (210) pour une future séance de sommeil sur la base au moins en partie d'une mesure de sommeil cible pour l'utilisateur (210) pour la future séance de sommeil, et éventuellement dans lequel la durée totale de sommeil estimée inclut une durée de sommeil de thérapie, une durée de sommeil hors thérapie, ou les deux, la durée de sommeil de thérapie étant une durée pendant laquelle l'utilisateur dort et est connecté au système de thérapie respiratoire (120) et la durée de sommeil hors thérapie étant une durée pendant laquelle l'utilisateur (210) ne reçoit pas l'air sous pression du système de thérapie respiratoire (120), et/ou dans lequel la mesure de sommeil cible pour l'utilisateur (210) inclut une mesure de sommeil de thérapie, une mesure de sommeil hors thérapie ou les deux.

9. Procédé selon l'une quelconque des revendications 1 à 8, dans lequel les données de traitement incluent des données physiologiques reçues du système de thérapie respiratoire (120), et le procédé comprend en outre :
la détermination d'une mesure de sommeil respiratoire sur la base des données physiologiques reçues du système de thérapie respiratoire (120) ; et
l'ajustement de la mesure de sommeil déterminée sur la base au moins en partie de la mesure de sommeil respiratoire, et éventuellement dans lequel les données physiologiques reçues du système de thérapie respiratoire (120) incluent un profil respiratoire de l'utilisateur, le profil respiratoire incluant une fréquence respiratoire, une profondeur respiratoire, une variabilité respiratoire ou toute combinaison de ces éléments.

10. Procédé selon l'une quelconque des revendications 1 à 9, comprenant en outre :
le déclenchement, par le dispositif électronique, de l'envoi d'une communication sur la base au moins en partie de la mesure de sommeil déterminée, et
éventuellement dans lequel la communication inclut la mesure de sommeil ; et/ou
dans lequel la communication inclut une recommandation à l'utilisateur (210) visant à : a) augmenter une durée de sommeil de thérapie par rapport à une durée de sommeil hors thérapie ; b) augmenter la durée de sommeil de thérapie par rapport à une durée de sommeil de thérapie antérieure ; ou c) les deux, la durée de sommeil de thérapie étant la durée pendant laquelle l'utilisateur dort et est connecté au système de thérapie respiratoire (120), et la durée de sommeil hors thérapie étant la durée pendant laquelle l'utilisateur (210) dort et ne reçoit pas l'air sous pression du système de thérapie respiratoire (120) ; ou
dans lequel la communication inclut une recommandation à l'utilisateur (210) visant à : (a) diminuer une durée de sommeil hors thérapie par rapport à une durée de sommeil de thérapie, (b) diminuer la durée de sommeil hors thérapie par rapport à une durée de sommeil hors thérapie antérieure, ou (c) les deux, la durée de sommeil de thérapie étant une durée pendant laquelle l'utilisateur (210) dort et est connecté au système de thérapie respiratoire (210) et la durée de sommeil hors thérapie étant une durée pendant laquelle l'utilisateur (210) dort et ne reçoit pas l'air sous pression du système de thérapie respiratoire (120), ou
dans lequel la communication inclut une mesure de sommeil de thérapie, une durée de sommeil de thérapie, une mesure de sommeil hors thérapie, une durée de sommeil hors thérapie, une mesure de sommeil estimée pour une future séance de sommeil, une mesure de sommeil cible pour la future séance de sommeil, une durée de sommeil cible pour la future séance de sommeil, une réduction cible de la durée de sommeil hors thérapie, ou une augmentation cible de la durée de sommeil de thérapie, ou toute combinaison de ces éléments.

11. Procédé selon la revendication 10, dans lequel la communication inclut une recommandation à l'utilisateur (210) visant à augmenter la durée totale de sommeil.

12. Procédé selon l'une quelconque des revendications 1 à 11, dans lequel un profil d'événements, un regroupement d'événements, ou les deux sont utilisés pour distinguer les données hors thérapie des données de thérapie ; et/ou
dans lequel un bruit associé à un moteur du système de thérapie respiratoire (120), un bruit associé à un mouvement de l'utilisateur (210) ou les deux sont utilisés pour distinguer les données hors thérapie des données de thérapie ; et/ou
dans lequel un bruit associé à la respiration de l'utilisateur (210) lorsque le système de thérapie respiratoire (120) est connecté à l'utilisateur (210) et fournit de l'air sous pression aux voies respiratoires de l'utilisateur (210), un bruit associé à la respiration de l'utilisateur (210) lorsque le système de thérapie respiratoire (120) ne fournit pas d'air sous pression aux voies respiratoires de l'utilisateur (210), ou les deux sont utilisés pour distinguer les données hors thérapie des données de thérapie.

13. Procédé selon l'une quelconque des revendications 1 à 12, comprenant en outre :
l'analyse de données audio générées par le capteur (130), pour identifier des caractéristiques indicatives d'une expiration par l'utilisateur (210), des caractéristiques indicatives d'une fuite d'une interface utilisateur (124) du système de thérapie respiratoire (120), ou les deux ;
dans lequel les caractéristiques indicatives de l'expiration par l'utilisateur (210) et de la fuite de l'interface utilisateur (124) du système de thérapie respiratoire (120), ou les deux, sont utilisées pour distinguer les données hors thérapie des données de thérapie, et
éventuellement dans lequel des données physiologiques générées lors de l'utilisation à la fois des caractéristiques indicatives de l'expiration par l'utilisateur (210) et des caractéristiques indicatives de la fuite de l'interface utilisateur (124) du système de thérapie respiratoire (120) sont incluses dans les données de thérapie.

14. Procédé selon l'une quelconque des revendications 1 à 13, comprenant en outre la génération de secondes données physiologiques relatives à une ou plusieurs comorbidités dont souffre l'utilisateur (210), et
éventuellement, dans lequel les secondes données physiologiques sont générées pendant la séance de sommeil ; et/ou
dans lequel l'incidence de la ou des comorbidités, l'évolution de la ou des comorbidités, ou les deux, sont corrélées avec une ou plusieurs des mesures suivantes : la mesure de sommeil, la mesure de sommeil de thérapie, la durée de sommeil de thérapie, la mesure de sommeil hors thérapie et la durée de sommeil hors thérapie.

15. Système comprenant :
un système de commande (110) incluant un ou plusieurs processeurs (112) ;
un capteur (130) ; et
une mémoire (114) sur laquelle sont stockées des instructions lisibles par machine ;
dans lequel le système de commande (110) est connecté à la mémoire (114), et le système est configuré pour mettre en œuvre le procédé selon l'une quelconque des revendications 1 à 14 lorsque les instructions lisibles par machine dans la mémoire (114) sont exécutées par au moins un du ou des processeurs (112) du système de commande (110).
